(19) **Europäisches Patentamt / European Patent Office / Office européen des brevets**

(11) **EP 4 350 311 B1**

(12) ## EUROPEAN PATENT SPECIFICATION

(45) Date of publication and mention of the grant of the patent:
**06.05.2026 Bulletin 2026/19**

(21) Application number: **23758894.2**

(22) Date of filing: **05.01.2023**

(51) International Patent Classification (IPC):
*G01K 13/20* (2021.01)  *G01K 17/00* (2006.01)
*H04R 1/10* (2026.01)  *G01K 1/20* (2006.01)
*G01K 1/14* (2021.01)  *G01K 1/16* (2006.01)
*G01K 1/02* (2021.01)  *A61B 5/01* (2006.01)
*A61B 5/00* (2006.01)

(52) Cooperative Patent Classification (CPC):
**G01K 13/20; A61B 5/01; A61B 5/6817; G01K 1/026; G01K 1/14; G01K 1/16; G01K 1/20; G01K 17/00; H04R 1/1016;** Y02D 10/00

(86) International application number:
**PCT/CN2023/070645**

(87) International publication number:
**WO 2023/160268 (31.08.2023 Gazette 2023/35)**

(54) **WEARABLE ELECTRONIC APPARATUS, BODY TEMPERATURE MEASUREMENT METHOD, AND WEARABLE ELECTRONIC DEVICE**

TRAGBARE ELEKTRONISCHE VORRICHTUNG, KÖRPERTEMPERATURMESSVERFAHREN UND TRAGBARE ELEKTRONISCHE VORRICHTUNG

APPAREIL ÉLECTRONIQUE PORTATIF, PROCÉDÉ DE MESURE DE TEMPÉRATURE CORPORELLE, ET DISPOSITIF ÉLECTRONIQUE PORTATIF

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **25.02.2022 CN 202210182919**

(43) Date of publication of application:
**10.04.2024 Bulletin 2024/15**

(73) Proprietor: **Honor Device Co., Ltd. Shenzhen, Guangdong 518040 (CN)**

(72) Inventors:
• **QI, Lipeng** Shenzhen, Guangdong 518040 (CN)
• **YAO, Chao** Shenzhen, Guangdong 518040 (CN)

• **LIU, Yi** Shenzhen, Guangdong 518040 (CN)
• **LI, Chenlong** Shenzhen, Guangdong 518040 (CN)

(74) Representative: **Epping - Hermann - Fischer Patentanwaltsgesellschaft mbH Schloßschmidstraße 5 80639 München (DE)**

(56) References cited:
CN-A- 112 577 611    CN-A- 113 167 657
CN-A- 113 473 298    CN-A- 113 473 298
CN-A- 114 052 669    CN-A- 114 052 669
CN-U- 213 029 911    CN-U- 215 414 054
KR-A- 20210 006 073   US-A1- 2016 331 244
US-A1- 2018 214 028   US-A1- 2019 117 155
US-A1- 2019 117 155   US-A1- 2022 047 174

## Description

This application claims priority to Chinese Patent Application No. 202210182919.1, filed with China National Intellectual Property Administration on February 25, 2022 and entitled "WEARABLE ELECTRONIC APPARATUS, BODY TEMPERATURE MEASUREMENT METHOD, AND WEARABLE ELECTRONIC DEVICE".

## TECHNICAL FIELD

This application relates to the field of electronic technologies, and in particular, to a wearable electronic apparatus, a body temperature measurement method, and a wearable electronic device.

## BACKGROUND

With the continuous development of science and technology, people have higher and higher functional requirements for earphones and the like.

Body temperature can reflect the human health condition, and a core temperature of the human body can best reflect the body temperature of the human body. In medicine, a temperature of the pulmonary artery is defined as the core temperature. Because the pulmonary artery is inside the human body, it is difficult to measure in conventional means. Currently, axillary temperature, rectal temperature, oral temperature, eardrum temperature, forehead temperature, and the like are usually used as substitute tests for the core temperature to measure human body temperature. Because the eardrum is close to the pulmonary artery, the eardrum temperature can more accurately reflect the core temperature of the human body than other substitute tests. In the related technologies, the eardrum temperature is mainly measured by an ear thermometer.

However, the earphones in the related technologies cannot measure the body temperature of the human body, and a function of the earphones is single.

US 2019/117155 A1 relates to an electronic device configured to measure temperature from within an ear canal of an ear comprising a first bend and a second bend, the device comprising: an enclosure configured for insertion into the ear canal and comprising a distal end configured to extend at least beyond the first bend; a distal temperature sensor situated at a location of the enclosure that faces a tragus-side of the ear canal distal to the first bend and proximal to the second bend when the enclosure is fully inserted into the ear canal, the distal temperature sensor configured to sense one or both of conductive heat and convective heat and to produce a first temperature signal; a proximal temperature sensor situated on the enclosure at a location spaced apart from a surface of the ear canal and proximal to the distal temperature sensor location in an outer ear direction when the enclosure is fully inserted into the ear canal, the proximal temperature sensor configured to sense one or both of conductive heat and convective heat and to produce a second temperature signal; a memory configured to store a pre-established heat balance equation; and a processor coupled to the distal and proximal temperature sensors and the memory, the processor configured to calculate an absolute core body temperature using the heat balance equation and the first and second temperature signals.

## SUMMARY

This application provides a wearable electronic apparatus, a body temperature measurement method, and a wearable electronic device, and a core temperature of a user can be measured. The invention is set out in the appended claims.

According to a first aspect of the invention, a wearable electronic apparatus according to claim 1 is provided.

The wearable electronic apparatus includes a housing, a processor, and a temperature sensor located in the housing. The housing has a sound outlet hole. The temperature sensor includes a first temperature sensor and a second temperature sensor. The first temperature sensor and the second temperature sensor respectively measure temperatures at different temperature measurement points.

The second temperature sensor is located on a side of the first temperature sensor opposite to the sound outlet hole. The processor is configured to determine a core temperature of a user based on a thermal equilibrium temperature corresponding to the first temperature sensor and a thermal equilibrium temperature corresponding to the second temperature sensor.

In this embodiment of this application, the first temperature sensor and the second temperature sensor are disposed in the wearable electronic apparatus. The first temperature sensor and the second temperature sensor can respectively measure temperatures at different temperature measurement points in the wearable electronic apparatus. Because a temperature is proportional to thermal resistance during a heat transfer process, the core temperature of the user can be determined based on the thermal equilibrium temperature corresponding to the first temperature sensor and the thermal equilibrium temperature corresponding to the second temperature sensor by provision of the processor. In this way, the core temperature of the user can be measured, and functions of the wearable electronic apparatus can be more diversified.

In a possible implementation, the temperature sensor further includes a third temperature sensor. The third temperature sensor is located on a side that the second temperature sensor is away from the first temperature sensor, to measure an ambient temperature in which the wearable electronic apparatus is located.

The processor is configured to determine the core temperature of the user based on the thermal equilibrium temperature corresponding to the first temperature sensor, the thermal equilibrium temperature corresponding to the second temperature sensor, and the ambient temperature.

In this way, by provision of the third temperature sensor, an impact of the ambient temperature on the core temperature can be eliminated, and accuracy of the wearable electronic apparatus for the core temperature measurement can be improved.

In a possible implementation, a part that is of the housing and that is located on the third temperature sensor is located outside an ear. In this way, accuracy for measuring the ambient temperature by the third temperature sensor is not only improved, but internal space of the housing also can be used effectively.

In a possible implementation, the wearable electronic apparatus includes a calculation module. The calculation module is located in the housing. The processor is configured to control the calculation module to determine the core temperature based on the thermal equilibrium temperature corresponding to the first temperature sensor, the thermal equilibrium temperature corresponding to the second temperature sensor, and the ambient temperature.

In this way, the core temperature can be calculated and determined by using the calculation module. In a possible implementation, the processor is configured to control the calculation module to determine the core temperature based on a formula:

$$\mathrm{T} = \frac{R_2(T_1 - T_2 - b \times T_3)}{R_1} + T_1 + a \times T_3,$$

where

T is the core temperature, $T_1$ is the thermal equilibrium temperature corresponding to the first temperature sensor, $T_2$ is the thermal equilibrium temperature corresponding to the second temperature sensor, $T_3$ is the ambient temperature, $R_1$ is equivalent thermal resistance between the first temperature sensor and the second temperature sensor during a heat transfer process, $R_2$ is equivalent thermal resistance between human tissue and the first temperature sensor during a heat transfer process, and a and b are both adjustment factors.

In this way, by provision of $T_3$, a, and b, an impact of an external influence factor and an impact of a thermal power consumption heat source of the wearable electronic apparatus on the core temperature can be eliminated, and accuracy of the wearable electronic apparatus for the core temperature measurement is ensured.

The housing has a contact part, the contact part is configured to be located in ear and in contact with the ear, and the first temperature sensor and the contact part are disposed corresponding to each other.

In this way, when the wearable electronic apparatus is worn on the ear of the user, a temperature of the ear can be transferred as much as possible to a position in which the first temperature sensor is located through the contact part, to reduce a difference between the thermal equilibrium temperature corresponding to the first temperature sensor and the temperature of the ear.

The wearable electronic apparatus includes a thermally conductive metal member. The thermally conductive metal member is embedded in the contact part and exposed on an outer surface of the housing.

By disposing the thermally conductive metal member, when the wearable electronic apparatus is worn on the ear of the user, the wearable electronic apparatus can directly be in contact with the ear of the user, and heat of the user is transferred to the position in which the first temperature sensor is located, to further reduce the difference between the thermal equilibrium temperature corresponding to the first temperature sensor and the temperature of the ear.

In a possible implementation, thermal resistance between the second temperature sensor and the ear is greater than thermal resistance between the first temperature sensor and the ear.

In this way, a temperature differential function of the second temperature sensor relative to the first temperature sensor can be implemented, so that the processor may determine the core temperature of the user based on the thermal equilibrium temperature corresponding to the first temperature sensor and the thermal equilibrium temperature corresponding to the second temperature sensor. In a possible implementation, the thermally conductive metal member is a stainless steel member, a copper member, or an aluminum member.

In this way, the thermal resistance between the first temperature sensor and the ear can be reduced, so that the thermal resistance between the second temperature sensor and the ear is greater than the thermal resistance between the first temperature sensor and the ear.

In a possible implementation, the thermally conductive metal member is connected to the housing in a sealing manner.

In this way, a sealing performance of the wearable electronic apparatus can be enhanced, further improving accuracy for measuring the core temperature.

In a possible implementation, a distance between the second temperature sensor and an eardrum of the user is greater than a distance between the first temperature sensor and the eardrum of the user.

In this way, the thermal resistance between the second temperature sensor and the ear can be increased.

The wearable electronic apparatus further includes a flexible circuit board located in the housing. Both the first temperature sensor and the second temperature sensor are located on the flexible circuit board.

In this way, by disposing the flexible circuit board, the first temperature sensor and the second temperature sensor not only can be fixed in the housing, but an internal signal of the wearable electronic apparatus also can be transmitted.

The first temperature sensor is located on a side that is of the flexible circuit board and faces to a center of the housing, and the first temperature sensor is configured to measure a temperature of a position that is on the flexible circuit board and that corresponds to the first temperature sensor. In this way, the first temperature sensor may measure the core temperature by measuring a temperature of the flexible circuit board, and internal space of the housing can also be used effectively.

The housing has a thermally conductive layer, and the thermally conductive layer is fitted between the flexible circuit board and the thermally conductive metal member on the housing.

By disposing the thermally conductive layer, heat transfer between the thermally conductive metal member and the flexible circuit board can be better implemented.

In a possible implementation, the housing includes an earphone handle housing and an earphone housing connected to the earphone handle housing in a detachable manner. The first temperature sensor and the second temperature sensor are located in the earphone housing, and the third temperature sensor is located in the earphone handle housing.

In this way, the first temperature sensor and the second temperature sensor can be closer to the ear of the user than the third temperature sensor, and the third temperature sensor is away from the ear, further improving accuracy for measuring the core temperature.

According to a second aspect of the invention, a body temperature measurement method according to claim 8 is provided.

The measurement method is applied to the wearable electronic apparatus according to any one of the above. The measurement method includes:

obtaining thermal equilibrium temperatures at different temperature measurement points of a housing in the wearable electronic apparatus, where the temperature measurement points include a first temperature measurement point and a second temperature measurement point, and the first temperature measurement point is located on a side of the second temperature measurement point facing the sound outlet hole of the wearable electronic apparatus; and determining a core temperature of the user based on the thermal equilibrium temperature at the first temperature measurement point and the thermal equilibrium temperature at the second temperature measurement point.

In this way, the wearable electronic apparatus can measure the core temperature of the user, and functions of the wearable electronic apparatus is more diversified.

Before the obtaining thermal equilibrium temperatures at different temperature measurement points of a housing in the wearable electronic apparatus, the measurement method further includes:
responding to a temperature measurement operation of the user.

Alternatively, after the determining a core temperature of the user based on the thermal equilibrium temperature at the first temperature measurement point and the thermal equilibrium temperature at the second temperature measurement point, the measurement method further includes:
outputting a temperature measurement result including the core temperature.

This not only facilitates the user to control the wearable electronic apparatus to measure the core temperature through the temperature measurement operation, but also facilitates the user to obtain the core temperature.

In a possible implementation, the temperature measurement points further include an ambient temperature measurement point, and the ambient temperature measurement point is located on a side that the second temperature measurement point is away from the first temperature measurement point; and
the determining a core temperature of the user based on the thermal equilibrium temperature at the first temperature measurement point and the thermal equilibrium temperature at the second temperature measurement point includes:
determining the core temperature of the user based on the thermal equilibrium temperature at the first temperature measurement point, the thermal equilibrium temperature at the second temperature measurement point, and an ambient temperature at the ambient temperature measurement point.

In this way, through the ambient temperature at the ambient temperature measurement point, an impact of an external influence factor on the core temperature can be eliminated, and accuracy of the wearable electronic apparatus for the core temperature measurement is improved.

In a possible implementation, before the obtaining thermal equilibrium temperatures at different temperature measurement points of a housing in the wearable electronic apparatus, the measurement method further includes: determining the ambient temperature at the ambient temperature measurement point.

In this way, through the ambient temperature at the ambient temperature measurement point, the core temperature can be corrected, and accuracy for calculating the core temperature is improved.

In a possible implementation, the determining the ambient temperature at the ambient temperature measurement point specifically includes:

obtaining an initial temperature at the ambient temperature measurement point, where the initial temperature is a temperature at the ambient temperature measurement point before the wearable electronic apparatus is worn on ear of the user;

obtaining a momentary temperature at the ambient temperature measurement point, where the momentary temperature is a temperature at the ambient temperature measurement point when the wearable electronic apparatus is worn on the ear of the user within a first preset time; and

determining the ambient temperature at the ambient temperature measurement point based on the initial temperature and the momentary temperature.

In this way, a preliminary determining of the ambient temperature can be made to eliminate an impact of a first type of factor, such as body temperature in a pocket of the user, charging, and a finger of user touching, on the measurement of the core temperature of the user, and improve accuracy of the wearable electronic apparatus for the core temperature measurement.

In a possible implementation, the determining the ambient temperature at the ambient temperature measurement point based on the initial temperature and the momentary temperature specifically includes: using a lower temperature between the initial temperature and the momentary temperature as the ambient temperature at the ambient temperature measurement point.

In this way, the ambient temperature at the ambient temperature measurement point can be determined based on changes of the initial temperature and the momentary temperature.

In a possible implementation, before the determining a core temperature of the user based on the thermal equilibrium temperature at the first temperature measurement point and the thermal equilibrium temperature at the second temperature measurement point, the measurement method further includes: correcting the ambient temperature at the ambient temperature measurement point based on a thermal equilibrium temperature at the ambient temperature measurement point.

This can facilitate to correct the ambient temperature at the ambient temperature measurement point through the thermal equilibrium temperature at the ambient temperature measurement point, and can eliminate an impact of the external influence factor on the ambient temperature at the ambient temperature measurement point.

In a possible implementation, the correcting the ambient temperature at the ambient temperature measurement point based on a thermal equilibrium temperature at the ambient temperature measurement point specifically includes:

detecting a working scenario of the wearable electronic apparatus, where the working scenario includes a low power consumption scenario, a medium power consumption scenario, and a high power consumption scenario;

measuring the thermal equilibrium temperatures at the temperature measurement points; and

determining an adjustment factor based on the working scenario and the thermal equilibrium temperature at the ambient temperature measurement point.

In this way, through the adjustment factor, an impact of a second type of factor, such as different power consumption working scenarios, on the measurement of the core temperature of the user can be eliminated, further improving accuracy of the wearable electronic apparatus for the core temperature measurement.

In a possible implementation, the correcting the ambient temperature at the ambient temperature measurement point based on a thermal equilibrium temperature at the ambient temperature measurement point specifically includes:

determining whether the thermal equilibrium temperature at the ambient temperature measurement point is the same as the ambient temperature at the ambient temperature measurement point;

determining the core temperature based on the ambient temperature at the ambient temperature measurement point if the thermal equilibrium temperature at the ambient temperature measurement point is the same as the ambient temperature at the ambient temperature measurement point; and

if the thermal equilibrium temperature at the ambient temperature measurement point is different from the ambient temperature at the ambient temperature measurement point, determining the ambient temperature at the ambient temperature measurement point as the thermal equilibrium temperature at the ambient temperature measurement point, and continuing to correct the ambient temperature at the ambient temperature measurement point until the thermal equilibrium temperature at the ambient temperature measurement point is the same as the determined ambient temperature at the ambient temperature measurement point.

In this way, through a change of the thermal equilibrium temperature at the ambient temperature measurement point relative to the ambient temperature at the ambient temperature measurement point, a third type of factor, such as a scenario that an ear is covered by an obstruction, can be judged, to avoid an impact of the third type of factor on the core temperature measurement of the user, further improving accuracy of the wearable electronic apparatus for the core temperature measurement.

In a possible implementation, the determining a core temperature of a user based on the thermal equilibrium temperature at the first temperature measurement point and the thermal equilibrium temperature at the second temperature measurement point specifically includes:

determining the core temperature of the user based on the thermal equilibrium temperature at the first temperature measurement point, the thermal equilibrium temperature at the second temperature measurement point, the ambient temperature at the ambient temperature measurement point, and the adjustment factor.

In this way, accuracy of a measurement result of the core temperature performed by the wearable electronic apparatus is high.

In a possible implementation, after the determining a core temperature of the user based on the thermal equilibrium temperature at the first temperature measurement point and the thermal equilibrium temperature at the second temperature measurement point, the measurement method further includes:

obtaining the thermal equilibrium temperatures at the different temperature measurement points of the housing in the wearable electronic apparatus;

determining whether the thermal equilibrium temperatures at the temperature measurement points change compared with the thermal equilibrium temperatures at the determined temperature measurement points; and

determining the core temperature of the user based on changed thermal equilibrium temperatures at the temperature measurement points if the thermal equilibrium temperature at the temperature measurement points change.

In this way, an impact of a fourth type of factor, such as a change in an indoor and outdoor scenario, on the core temperature of the user can be eliminated, and the measurement result of the core temperature is improved.

In a possible implementation, after the determining a core temperature of the user based on the thermal equilibrium temperature at the first temperature measurement point and the thermal equilibrium temperature at the second temperature measurement point, the measurement method further includes:

determining whether the wearable electronic apparatus is worn on the ear of the user again within a second preset time; and

if the wearable electronic apparatus is worn on the ear of the user again within the second preset time, determining the core temperature of the user based on the ambient temperature at the ambient temperature measurement point before the wearable electronic apparatus is put off. This can avoid that when the wearable electronic apparatus is put on and off for a short period of time, the ambient temperature at the ambient temperature measurement point in the housing fails to drop to an actual ambient temperature, and measurement of the core temperature is affected, and further improve accuracy of the wearable electronic apparatus for the core temperature measurement.

According to a third aspect, an embodiment of this application provides a wearable electronic device. The wearable electronic device includes a charging accommodating apparatus and a wearable electronic apparatus according to any one of the apparatus provided in the first aspect. The wearable electronic apparatus is disposed in the charging accommodating apparatus.

In this way, by disposing the charging accommodating apparatus, the wearable electronic apparatus not only can be charged, but the wearable electronic apparatus also can be accommodated.

According to a fourth aspect, a storage medium according to claim 15 is provided. The storage medium stores computer-executable instructions. When the computer-executable instructions are executed by the processor of an apparatus provided in the first aspect, the body temperature measurement method according to any one of the above is implemented.

In this way, by provision of the computer-executable instructions stored in the storage medium, the processor is enabled to perform the body temperature measurement method according to embodiments of this application based on the computer-executable instructions, to measure the core temperature of the user.

## BRIEF DESCRIPTION OF DRAWINGS

FIG. 1 is a schematic diagram of a structure of an earphone according to an embodiment of this application;

FIG. 2 is a schematic partial view of an earphone according to an embodiment of this application;

FIG. 3 is a schematic diagram of a temperature measurement model of an earphone according to an embodiment of this application;

FIG. 4 is a first schematic diagram of a temperature measurement principle of an earphone according to an embodiment of this application;

FIG. 5 is a second schematic diagram of a temperature measurement principle of an earphone according to an embodiment of this application;

FIG. 6 is a schematic diagram of an internal structure of the earphone in FIG. 2 according to an embodiment of this application;

FIG. 7 is a schematic diagram of an internal structure of an earbud of the earphone in FIG. 6;

FIG. 8 is an enlarged view of a part A of the earphone in FIG 7;

FIG. 9 is a schematic diagram of an assembly of a first temperature sensor in an earphone housing according to an embodiment of this application;

FIG. 10 is a schematic flowchart of a body temperature measurement method according to an embodiment of this application;

FIG. 11 is a schematic flowchart of another body temperature measurement method according to an embodiment of this application;

FIG. 12 is a schematic diagram of an impact of a first type of factor on an ambient temperature at a temperature measurement zone in an earphone according to an embodiment of this application;

FIG. 13 is a simulated view that an obstruction covers a head phantom according to an embodiment of this application;

FIG. 14 is a schematic diagram of an impact of a fourth type of factor on ambient temperature at a first temperature measurement zone in an earphone according to an embodiment of this application;

FIG. 15 is a schematic flowchart of determining ambient temperature at an ambient temperature measurement zone according to an embodiment of this application;

FIG. 16 is a schematic flowchart of correcting thermal equilibrium temperature of an earphone at different temperature measurement zones according to an embodiment of this application;

FIG. 17 is a schematic flowchart of still another body temperature measurement method according to an embodiment of this application;

FIG. 18 is a schematic flowchart of yet another body temperature measurement method according to an embodiment of this application; and

FIG. 19 is a schematic flowchart of still yet another body temperature measurement method according to an embodiment of this application.

Descriptions of reference numerals:

100: wearable electronic apparatus; 10: housing; 11: earphone housing; 111: first temperature measurement point; 1111: contact part; 1112: mounting hole; 112: second temperature measurement point; 113: sound outlet hole; 12: earphone handle housing; 121: ambient temperature measurement points;

20: temperature sensor; 21: first temperature sensor; 22: second temperature sensor; 23: third temperature sensor; 30: processor; 40: speaker; 50: thermally conductive metal member; 60: thermally conductive layer; 70: sealing member; 80: flexible circuit board;

200: human body heat source; 300: ambient heat source; 400: power consumption heat source; 500: ear; 600: obstruction.

## DESCRIPTION OF EMBODIMENTS

Terms used in description of this application are merely intended to explain specific embodiments of this application rather than limit this application.

Core temperature: Refers to a temperature of the pulmonary artery in medicine.

Thermal equilibrium: Refers to a situation that an internal temperature of an object in contact with the outside world is even everywhere and equal to outside temperature. In thermal equilibrium, there is no heat exchange between parts of the object and between the object and the outside world. Thermal equilibrium temperature: Refers to a temperature inside or on a surface of the wearable electronic apparatus, when an object such as a wearable electronic apparatus and ears of a user are in a thermal equilibrium process.

Body temperature is one of the basic health characteristics of human body. The body temperature changes depending on gender, age, time of day, level of exertion, health conditions (such as illness and menstruation), state of consciousness (such as waking, sleeping, and sedation), and mood. By measuring the body temperature, health conditions of people may be accurately monitored to a certain extent, so that people may understand their own health conditions, provide early warning of high temperature and heat stroke, assist in the diagnosis of illness, recall a course of disease, perform high and low temperature protection during exercise, and the like.

The core temperature of the human body can best reflect the body temperature of the human body. Because the pulmonary artery is inside the human body, it is difficult to measure in a conventional manner. Currently, axillary temperature, rectal temperature, oral temperature, eardrum temperature, forehead temperature, and the like for the measurement of human body temperature are usually used as substitute test quantities for the core temperature to measure the human body temperature. Because the eardrum is close to the pulmonary artery, the eardrum temperature can more accurately reflect the core temperature than other substitute tests.

Currently, a medical ear thermometer may be used for measuring an eardrum temperature. The medical ear thermometer in the related technologies usually use an infrared thermopile sensor to implement eardrum temperature measurement.

However, volume of the infrared thermopile sensor is large and difficult to integrate into the wearable electronic apparatus such as earphones, and cannot achieve an automatic and continuous measurement of the human body temperature. Currently, wearable electronic apparatuses cannot measure the human body temperature such as the eardrum temperature or the core temperature.

In view of this, embodiments of this application provide a wearable electronic apparatus, a body temperature measurement method, and a wearable electronic device. By disposing a first temperature sensor and a second temperature sensor in the wearable electronic apparatus, thermal equilibrium temperatures at different temperature measurement points of a housing can be measured. Because a temperature is proportional to thermal resistance during a heat transfer process, a core temperature of a user can be determined based on a thermal equilibrium temperature corresponding to the first temperature sensor and a thermal equilibrium temperature corresponding to the second temperature sensor by provision of a processor. In this way, the core temperature of the user can be measured, and diversity of functions of the wearable electronic apparatus can be increased.

The wearable electronic apparatus may include, but is not limited to, earphones or another electronic device that can be worn on ears of the user. The earphones may include but are not limited to wireless earphones.

The following further describes a structure of the wearable electronic apparatus in this embodiment of this application by using the wireless earphones as an example.

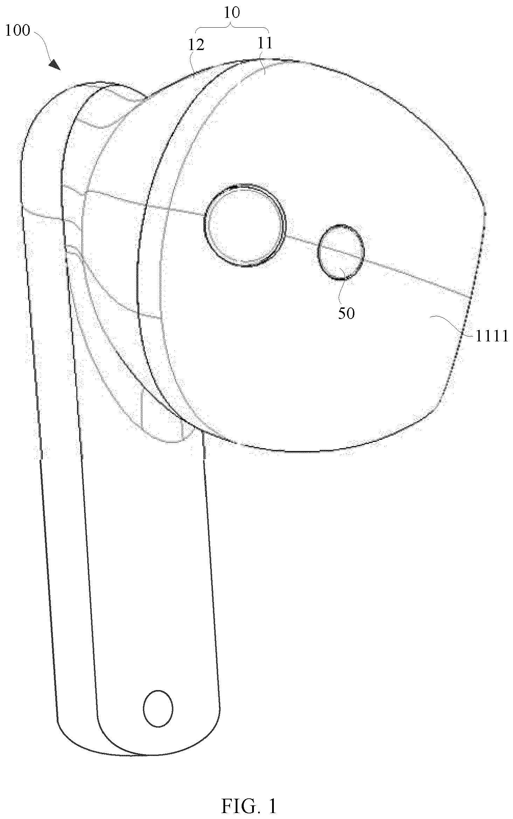

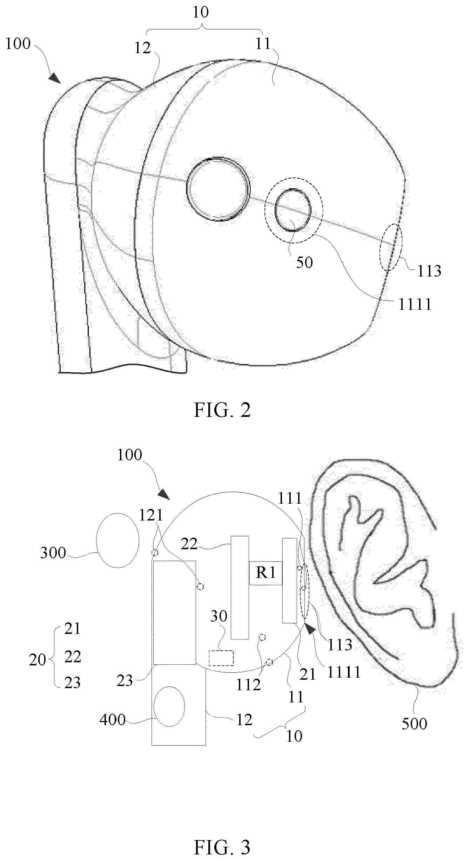

FIG. 1 and FIG. 2 are schematic diagrams of an integral and partial structure of an earphone.

As shown in FIG. 1 and FIG. 2, a wearable electronic apparatus 100 such as an earphone may include a housing 10, and the housing 10 may include but is not limited to a plastic housing. The housing 10 has a cavity to facilitate provision of electronic components inside the earphone. The housing 10 may include an earphone handle housing 12 and an earphone housing 11. The earphone housing 11 is connected to the earphone handle housing 12 in a detachable manner, and forms the cavity together with the earphone handle housing 12 for provision of the electronic components inside the wearable electronic apparatus 100 such as a speaker and a flexible circuit board. Alternatively, in some embodiments, the earphone housing 11 may also be integrally connected to the earphone handle housing 12. In this embodiment, a connection manner between the earphone housing 11 and the earphone handle housing 12 is not further limited. When the wearable electronic apparatus 100 is worn on an ear 500 of a user, the earphone handle housing 12 may be located outside the ear 500, the earphone housing 11 is located on a side that the earphone handle housing 12 faces to the ear 500 of the user, and at least a part of a structure is located in the ear 500. As shown in FIG. 2, the housing 10 has a sound outlet hole 113. The sound outlet hole 113 may be located on a side that is of the earphone housing 11 and that is away from the earphone handle housing 12, so that a sound signal of the earphone may be transmitted to the ear 500 of the user through the sound outlet hole 113.

It should be noted that, in some embodiments, the wearable electronic apparatus 100 may also include a flexible cap sleeved on the earphone housing 11. The flexible ear tip may be configured to be located in the ear 500. The flexible ear tip may be made of materials such as rubber or silicone, to improve comfort of the user when the wearable electronic apparatus 100 is worn.

FIG. 3 is a schematic diagram of a temperature measurement model of an earphone.

To implement a wearable electronic apparatus 100 to measure temperature of a user, the wearable electronic apparatus 100 has a plurality of temperature measurement points. FIG. 3 shows possible provision positions of the temperature measurement points in the wearable electronic apparatus 100.

As shown in FIG. 3, the temperature measurement points may include first temperature measurement points 111 and second temperature measurement points 112. The first temperature measurement points 111 and the second temperature measurement points 112 are located at different positions of the wearable electronic apparatus 100. The first temperature measurement points 111 may be located on a side that the second temperature measurement points 112 face to a sound outlet hole 113. In this way, the first temperature measurement points 111 is closer to an eardrum of the user than the second temperature measurement points 112, so that when the wearable electronic apparatus 100 is worn in an ear 500 of the user and heat of the user is transferred to the first temperature measurement points 111 and the second temperature measurement points 112, the first temperature measurement points 111 and the second temperature measurement points 112 have a certain temperature difference, thereby achieving measurement of body temperature of the user based on a proportional relationship between temperature and a thermal resistance during a heat transfer process.

As shown in FIG. 3, the first temperature measurement points 111 may be located on an earphone housing 11 or another electronic component in the earphone housing 11. In this way, when the wearable electronic apparatus 100 is worn on the ear 500 of the user, a distance between the first temperature measurement points 111 and the eardrum can be reduced, a loss in a process of heat of the user being transferred to the first temperature measurement points 111 can be reduced, and a difference between the first temperature measurement points 111 and body temperature of the user can be reduced.

In this embodiment, the second temperature measurement points 112 may also be located on the earphone housing 11 or another electronic component in a housing 10. In this way, while ensuring that the temperatures at the second temperature measurement point 112 and the first temperature measurement points 111 have a certain temperature difference and reducing a loss in a process of the heat of the user being transferred to the second temperature measurement points 112, space inside the earphone housing 11 can be properly used.

The housing 10 has a contact part 1111. A part that is of the housing 10 such as the earphone housing 12 and that corresponds to the first temperature measurement points 111 is located may constitute the contact part 1111, and the contact part 1111 is configured to be located in the ear 500 of the user, and in contact with the ear 500. When the first temperature measurement points 111 may be located on the earphone housing 11, the first temperature measurement points 111 may also be located in the contact part 1111.

It should be noted that, when the second temperature measurement points 112 is located on the earphone housing 11, and when the wearable electronic apparatus 100 is worn on the ear 500 of the user, a part that is of the housing 10 and that is located at the second temperature measurement points 112 may be in contact with the ear 500. Alternatively, in some embodiments, the part that is of the housing 10 and that is located at the second temperature measurement points 112 may also be located outside the ear 500 (in other words, not in contact with the ear 500). In this way, a provision position of the second temperature measurement points 112 can be more diversified. As shown in FIG. 3, the wearable electronic apparatus 100, such as the earphone further includes a temperature sensor 20. The temperature sensor 20 may be located in the housing 10. In this embodiment, the temperature sensor 20 may be a contact sensor. Because the contact sensor has smaller volume compared to an infrared thermopile sensor, the temperature sensor 20 can be laid out in effective cavity space of the earphone, so that the temperature sensor 20 can be integrated into the wearable electronic apparatus 100, such as the earphone, and the temperatures at different temperature measurement points are measured, to implement continuous measurement of a core temperature of the user by the wearable electronic apparatus 100.

As shown in FIG. 3, the temperature sensor 20 may include a first temperature sensor 21 and a second temperature sensor 22. The first temperature sensor 21 and the second temperature sensor 22 respectively measure temperatures at different temperature measurement points. The first temperature sensor 21 is configured to measure the temperatures at the first temperature measurement points 111. The first temperature sensor 21 and the contact part 111 may be disposed corresponding to each other. Alternatively, when a measurement end of the first temperature sensor 21 is exposed on the housing 10 such as the contact part 111, the measurement end may also be understood as the first temperature measurement points 111. For example, the first temperature sensor 21 may include but not limited to a contact temperature sensor. In this way, when the wearable electronic apparatus 100 such as the earphone is worn on the ear 500 of the user, the heat of the user may be transferred to the first temperature measurement points 111 through the contact part 1111, and the temperature at the first temperature measurement points 111, such as a thermal equilibrium temperature, may be measured by using the first temperature sensor 21, to facilitate to measure the body temperature of the user. In a thermal equilibrium process, heat transfer between the ear 500 and the wearable electronic apparatus 100 no longer occurs, and temperatures measured by the first temperature sensor 21 and the second temperature sensor 22 tend to be equilibrated. The second temperature sensor 22 is configured to measure the temperature at the second temperature measurement points 112. The second temperature sensor 22 may include, but is not limited to a contact temperature sensor, and the temperature sensor is a temperature difference sensor, to facilitate to implement a temperature difference function of the second temperature sensor 22 relative to the first temperature sensor 21. In this way, the temperatures at the second temperature measurement points 112, such as the thermal equilibrium temperature, may be measured by using the second temperature sensor 22, so that measurement of the temperatures at the different temperature measurement points in the wearable electronic apparatus 100, such as the first temperature measurement point 111 and the second temperature measurement points 112, is implemented based on the first temperature sensor 21 and the second temperature sensor 22, thereby implementing the measurement of the body temperature of the user.

As shown in FIG. 3, the wearable electronic apparatus 100 has a processor 30. The processor 30 is located in the housing 10. The processor 30 may be located in the earphone housing 11 or the earphone handle housing 12. In this embodiment, a provision position of the processor 30 in the wearable electronic apparatus 100 is not further limited. The processor 30 is configured to determine the core temperature of the user based on a thermal equilibrium temperature corresponding to the first temperature sensor 21 and a thermal equilibrium temperature corresponding to the second temperature sensor 22. The thermal equilibrium temperature corresponding to the first temperature sensor 21 may be understood as thermal equilibrium temperatures at the first temperature measurement points 111, and the thermal equilibrium temperature corresponding to the second temperature sensor 22 may be understood as thermal equilibrium temperatures at the second temperature measurement points 112. In this way, the core temperature of the user can be determined based on the thermal equilibrium temperature at the first temperature measurement points 111 and the thermal equilibrium temperatures at the second temperature measurement points 112 by provision of the processor 30, so that the core temperature of the user can be measured, to monitor a health condition of the user, and meanwhile functions of the wearable electronic apparatus 100 can be more diversified.

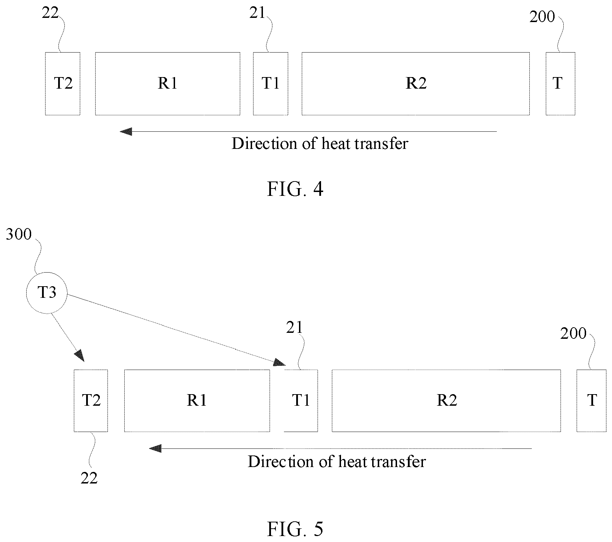

FIG. 4 is a schematic diagram of a temperature measurement principle of an earphone.

As shown in FIG. 4, when a wearable electronic apparatus 100 is worn on an ear 500 of a user, heat generated by a human body heat source 200 may be transferred to a first temperature sensor 21 and a second temperature sensor 22 in the wearable electronic apparatus 100. According to a relevant principle of heat transfer, during a heat transfer process, there is equivalent thermal resistance between the first temperature sensor 21 and the second temperature sensor 22 in the wearable electronic apparatus 100, such as the earphone, and there is equivalent thermal resistance between human tissue and the first temperature sensor 21. During the heat transfer process, a temperature and thermal resistance are proportional to each other. In a process that the human body heat source 200 may be transferred to the first temperature sensor 21 and the second temperature sensor 22, a thermal equilibrium temperature at a first temperature measurement point 111, a thermal equilibrium temperature at a second temperature measurement point 112, the equivalent thermal resistance between the first temperature sensor 21 and the second temperature sensor 22, and the equivalent thermal resistance between the human tissue and the first temperature sensor 21 may comply with a formula:

$$\frac{T-T_1}{R_1} = \frac{T_1-T_2}{R_1} \qquad \text{(Formula 1)}.$$

In the formula, $T$ is a core temperature, $T_1$ is a thermal equilibrium temperature corresponding to the first temperature sensor 21, $T_2$ is a thermal equilibrium temperature corresponding to the second temperature sensor 22, $R_1$ is the equivalent thermal resistance between the first temperature sensor 21 and the second temperature sensor 22 during the heat transfer process, and $R_2$ is the equivalent thermal resistance between the human tissue and the first temperature sensor 21 during the heat transfer process.

In this way, a processor 30 in an embodiment of this application may determine the core temperature $T$ of the user based on $T_1$, $T_2$, $R_1$, and $R_2$.

The processor 30 is configured to determine the core temperature of the user based on a formula

$$T = \frac{R_2(T_1-T_2)}{R_1} + T_1 \qquad \text{(Formula 2)},$$

thereby achieving measurement of the core temperature of the user, so that the user can learn about a health condition of the user at any time.

It should be noted that, before measuring the core temperature $T$ of the user, and when heat is transferred between the first temperature sensor 21 and the second temperature sensor 22 in a manner of thermal conduction, the equivalent thermal resistance $R_1$ between the first temperature sensor 21 and the second temperature sensor 22 may be determined in a manner of model simulation.

In this embodiment, before measuring the body temperature of the user by using the wearable electronic apparatus 100, eardrum temperature of the user may first be measured by a medical ear thermometer, and the core temperature $T$ may be replaced by the measured eardrum temperature, and then the wearable electronic apparatus 100 is worn on the ear 500 of the user, and an equivalent thermal resistance R_2 may be determined based on the first temperature sensor 21, the second temperature sensor 22, and an equivalent thermal resistance R_1. In this way, when measuring the temperature of the user by using the wearable electronic apparatus 100, the core temperature of the user may be determined based on the determined equivalent thermal resistance R_1 and the equivalent thermal resistance R_2.

Because the gender, height, or weight of the user may affect the equivalent thermal resistance R_2, when determining the equivalent thermal resistance R_2, users of different genders, height, or weight may be selected. After determining a plurality of initial equivalent thermal resistance R_2, the equivalent thermal resistance R_2 may be determined based on an average value of the plurality of initial equivalent thermal resistance R_2.

It should be noted that, because the eardrum temperature can more accurately reflect the core body temperature compared to the foregoing other alternative tests, in this embodiment, the eardrum temperature may be used for reflecting the core temperature of the user in replacement of the core temperature.

The wearable electronic apparatus 100 may further include a calculation module, and the calculation module may be electrically connected to the processor 30. The processor 30 is configured to control the calculation module to determine the core temperature based on the thermal equilibrium temperature corresponding to the first temperature sensor 21 and the thermal equilibrium temperature corresponding to the second temperature sensor 22. In this way, the core temperature may be calculated based on formula 2 by using the calculation module, to enable the processor 30 to determine the core temperature.

FIG. 5 is schematic diagram of another temperature measurement principle of an earphone.

As shown in FIG. 3 and FIG. 5, because the thermal equilibrium temperature corresponding to the first temperature sensor 21 and the thermal equilibrium temperature corresponding to the second temperature sensor 22 are affected by external environment, such as an ambient heat source 300, and thermal power consumption of the wearable electronic apparatus 100, such as a power consumption heat source 400, a core temperature measurement result may be affected.

To improve a measurement result of the core temperature, in some embodiments, the temperature measurement points may also include an ambient temperature measurement point 121. The ambient temperature measurement point 121 may be located on a side that the second temperature measurement point 112 is away from the first temperature measurement point 111 (as shown in FIG. 3). In this embodiment, the ambient temperature measurement point 121 may be located on the housing 10 or an electronic component in the housing 10. For example, the ambient temperature measurement point 121 may be located on the earphone handle housing 12 or an electronic component in the earphone handle housing 12. When the ambient temperature measurement point 121 may be located on the earphone handle housing 12, a part that is of the earphone handle housing 12 and that is at the ambient temperature measurement point 121 may be located outside the ear 500. This not only can make the ambient temperature measurement point 121 away from the ear 500 of the user, so that temperature at the ambient temperature measurement point 121 reflects a temperature in which the wearable electronic apparatus 100 is located, and the thermal equilibrium temperature corresponding to the first temperature sensor 21 and the thermal equilibrium temperature corresponding to the second temperature sensor 22 are corrected based on the temperature at the ambient temperature measurement point 121, and the core temperature measurement result can be improved, but internal space of the housing 10 also can be effectively used, to avoid provision of the temperature sensor 20 affects a size of the wearable electronic apparatus 100.

To facilitate measuring a temperature at the ambient temperature measurement point 121, the temperature sensor 20 may also include a third temperature sensor 23, for example, the third temperature sensor 23 may include, but is not limited to, a contact temperature sensor. The third temperature sensor 23 may be electrically connected to the processor 30. The third temperature sensor 23 is located on a side that the second temperature sensor 22 is away from the first temperature sensor 21, to measure an ambient temperature in which the third temperature sensor 23 is located, so that the ambient temperature in which the third temperature sensor 23 is located reflects the temperature in which the wearable electronic apparatus 100 is located. The ambient temperature in which the third temperature sensor 23 is located may be understood as the ambient temperature at the ambient temperature measurement point 121. The thermal equilibrium temperature at the ambient temperature measurement point 121 may be regarded as a special ambient temperature.

The processor 30 is configured to determine the core temperature of the user based on the thermal equilibrium temperature corresponding to the first temperature sensor 21, the thermal equilibrium temperature corresponding to the second temperature sensor 22, and the ambient temperature in which the third temperature sensor 23 is located. Specifically, the processor 30 may be configured to control a calculation module to determine the core temperature based on the thermal equilibrium temperature corresponding to the first temperature sensor 21, the thermal equilibrium temperature corresponding to the second temperature sensor 22, and the ambient temperature in which the third temperature sensor 23 is located, so that the core temperature is determined. In this way, by provision of the third temperature sensor 23, the thermal equilibrium temperature corresponding to the first temperature sensor 21 and the thermal equilibrium temperature corresponding to the second temperature sensor 22 can be corrected by using the ambient temperature in which the third temperature sensor 23 is located, to eliminate an impact of an external affecting factor on the core temperature, and improve accuracy of the wearable electronic apparatus 100 for a core temperature measurement.

The ambient temperature in which the third temperature sensor 23 is located may correct the core temperature based on a formula:

$$\frac{T - T_1 - a \times T_3}{R_1} = \frac{T_1 - T_2 - b \times T_3}{R_1} \qquad \text{(Formula 3)}.$$

In the formula, $T_3$ is the ambient temperature in which the third temperature sensor 23 is located, and $a$ and b are both adjustment factors.

In some embodiments, the processor is configured to determine the core temperature of the user based on a formula:

$$T = \frac{R_2(T_1 - T_2 - b \times T_3)}{R_1} + T_1 + a \times T_3 \qquad \text{(Formula 4)}.$$

It should be noted that, the calculation module may calculate the core temperature based on formula 4, to enable the processor 30 to determine the core temperature.

A working scenario of the wearable electronic apparatus 100 may include a low power consumption scenario, a medium power consumption scenario, and a high power consumption scenario. For example, the low power consumption scenario may include a scenario that the wearable electronic apparatus 100, such as an earphone, only be worn on the ear 500 of the user without any functional operation; the medium power consumption scenario may include, but is not limited to, a scenario that the earphone is worn on the ear 500 of the user, a scenario of playing music or noise reduction, and the like; and the high power consumption scenario may include, but is not limited to, a scenario of making a call by using the earphone, and the like.

A register is also disposed in the housing 10 of the wearable electronic apparatus 100. The register may be electrically connected with the processor 30. The processor 30 may control the register to detect a working scenario of the wearable electronic apparatus 100 (such as only wearing on the ear 500, playing music, noise reduction or call, and the like), to determine that the working scenario of the wearable electronic apparatus 100 is the low power consumption scenario, the medium power consumption scenario, or the high power consumption scenario.

The working scenario of the wearable electronic apparatus 100 is different, generated power consumption also changes correspondingly, and the thermal equilibrium temperature corresponding to the first temperature sensor 21 and the thermal equilibrium temperature corresponding to the second temperature sensor 22 also have different degrees of impact s. Therefore, by provision of $T_3$, $a$, and $b$, the impact of the external influence factor and an impact of thermal power consumption of the wearable electronic apparatus 100 on the core temperature can be eliminated as much as possible, the core temperature can be corrected, and accuracy of the wearable electronic apparatus 100 for a core temperature measurement can be ensured.

In this embodiment, $a$ and $b$ may be determined based on the working scenario of the wearable electronic apparatus 100 such as the earphone, a thermal equilibrium temperature in which the third temperature sensor 23 is located, and the thermal equilibrium temperatures at the ambient temperature measurement point 121. The thermal equilibrium temperature in which the third temperature sensor 23 is located may be understood as a special ambient temperature. The thermal equilibrium temperature in which the third temperature sensor 23 is located may be understood as the thermal equilibrium temperature at the ambient temperature measurement point.

Table 1 shows a change of the adjustment factor with the working scenario and the thermal equilibrium temperature in which the third temperature sensor is located.

| Adjustment factor | | Working scenario of the wearable electronic apparatus | | |
|---|---|---|---|---|
| | | Low power consumption scenario | Medium power consumption scenario | High power consumption scenario |
| Thermal equilibrium temperature in which the third | Low temperature range | $a_{11}\ b_{11}$ | $a_{21}\ b_{21}$ | $a_{31}\ b_{31}$ |
| | Medium temperature range | $a_{12}\ b_{12}$ | $a_{22}\ b_{22}$ | $a_{32}\ b_{32}$ |
| | High temperature | $a_{13}\ b_{13}$ | $a_{23}\ b_{23}$ | $a_{33}\ b_{33}$ |
| temperature sensor is lo- cated | range | | | |

$a_{11}$ and $b_{11}$, $a_{12}$ and $b_{12}$, and $a_{13}$ and $b_{13}$ respectively represent values corresponding to adjustment factors $a$ and $b$ when the thermal equilibrium temperature in which the third temperature sensor 23 is located is in the low temperature range, medium temperature range and high temperature range in the low power consumption scenario. $a_{21}$ and $b_{21}$, $a_{22}$ and $b_{22}$, and $a_{23}$ and $b_{23}$ respectively represent values corresponding to the adjustment factors $a$ and $b$ when the thermal equilibrium temperature in which the third temperature sensor 23 is located is in the low temperature range, medium temperature range and high temperature range in the medium power consumption scenario. $a_{31}$ and $b_{31}$, $a_{32}$ and $b_{32}$, and $a_{33}$ and $b_{33}$ respectively represent values corresponding to the adjustment factors $a$ and $b$ when the thermal equilibrium temperature in which the third temperature sensor 23 is located is in the low temperature range, medium temperature range and high temperature range in the high power consumption scenario. The low temperature range, the medium temperature range, and the high temperature range correspond to different temperature ranges.

For example, a temperature range corresponding to the low temperature range may include, but is not limited to, less than 15°C, a temperature range corresponding to the medium temperature range may include, but is not limited to, greater than or equal to 15°C, and less than or equal to 30°C, and a temperature range corresponding to the high temperature range may include, but is not limited to, greater than 30°C. The medium temperature range may also be a normal temperature range.

In this embodiment, when the core temperature being calculated specifically, the value of the adjustment factors $a$ and $b$ may be determined based on the thermal equilibrium temperature in which the third temperature sensor 23 is located and the working scenario of the wearable electronic apparatus 100.

For example, when the register detects that the wearable electronic apparatus 100, such as the earphone, is only worn on the ear 500 of the user and does not perform any functional operation, the register may determine that the wearable electronic apparatus 100 is in the low power consumption scenario; and when the measured thermal equilibrium temperature in which the third temperature sensor 23 is located is 14°C, and because 14°C is in a range of low temperature range, $a_{11}$ may be used as the adjustment factor a, and $b_{11}$ may be used as the adjustment factor b to determine the core temperature of the user.

For example, when the register detects that the wearable electronic apparatus 100, such as the earphone, is worn on the ear 500 of the user and plays music, the register may determine that the wearable electronic apparatus 100 is in the medium power consumption scenario; and when the measured thermal equilibrium temperature in which the third temperature sensor 23 is located is 25°C, and because 25°C is in a range of medium temperature range, $a_{22}$ may be used as the adjustment factor a, and $b_{22}$ may be used as the adjustment factor b to determine the core temperature of the user.

For example, when the register detects that the wearable electronic apparatus 100, such as the earphone, is worn on the ear 500 of the user and makes a call, the register may determine that the wearable electronic apparatus 100 is in the high power consumption scenario; and when the measured thermal equilibrium temperature in which the third temperature sensor 23 is located is 32°C, and because 32°C is in a range of high temperature range, $a_{33}$ may be used as the adjustment factor a, and $b_{33}$ may be used as the adjustment factor b to determine the core temperature of the user.

It should be noted that, in some embodiments, a temperature range corresponding to the thermal equilibrium temperature in which the third temperature sensor 23 is located may also be further refined, so that a quantity of the temperature range of the thermal equilibrium temperature in which the third temperature sensor 23 is located is greater than three.

In this embodiment, thermal resistance between the second temperature sensor 22 and the ear 500 may be greater than thermal resistance between the first temperature sensor 21 and the ear 500. In this way, a temperature differential function of the second temperature sensor 22 relative to the first temperature sensor 21 can be implemented, so that the processor 30 may determine the core temperature of the user based on the thermal equilibrium temperature corresponding to the first temperature sensor 21 and the thermal equilibrium temperature corresponding to the second temperature sensor 22.

The following uses a specific internal structure of the wearable electronic apparatus 100, to further explain specific positions of the temperature sensor 20 and the temperature measurement point.

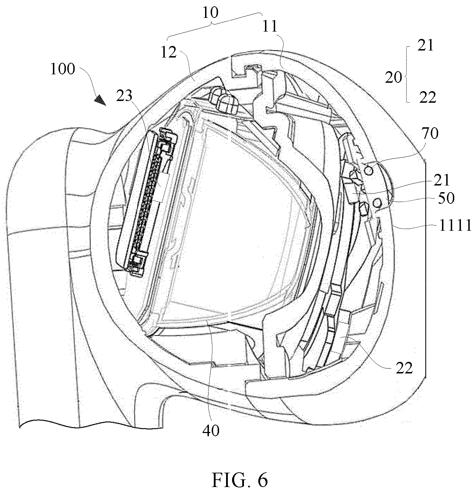

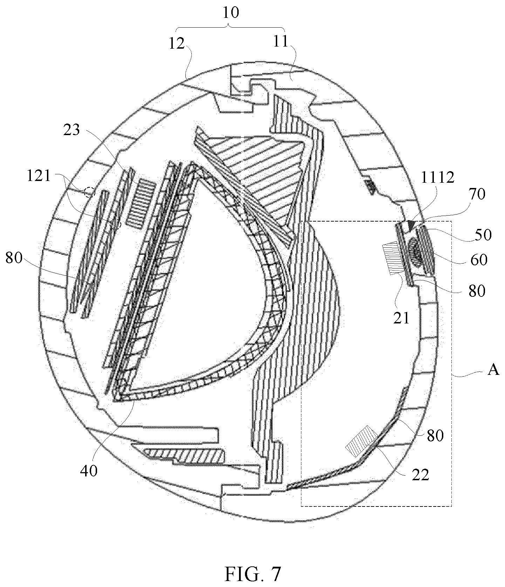

FIG. 6 is a schematic diagram of an internal structure of the earphone in FIG. 2. FIG. 7 is a schematic diagram of an internal structure of an earbud of the earphone in FIG. 6. FIG. 7 hides a part of the internal structure of the earphone to simplify the schematic diagram of the structure of the earphone.

As shown in FIG. 6 and FIG. 7, a first temperature sensor 21 and a second temperature sensor 22 may be located in the earphone housing 11, and a third temperature sensor 23 may be located in the earphone handle housing 12. As shown in FIG. 7, the wearable electronic apparatus 100 may further include a flexible circuit board 80 and a speaker 40, both the flexible circuit board 80 and the speaker 40 may be disposed in the housing 10, and the flexible circuit board 80 and the speaker 40 may be understood as electronic components in the housing 10. The flexible circuit board 80 may extend from the earphone housing 11 to a bottom of the earphone handle housing 12 for transmission of a signal inside the wearable electronic apparatus 100. The speaker 40 may be disposed on the flexible circuit board 80, and at least a part of a structure of the speaker 40 may be located in the earphone housing 11, so that an electrical signal is converted into an acoustic signal, and the acoustic signal is transmitted to the ear 500 of the user through the sound outlet hole 113.

The first temperature sensor 21, the second temperature sensor 22, and the third temperature sensor 23 may be disposed on the flexible circuit board 80 to fix the first temperature sensor 21, the second temperature sensor 22, and the third temperature sensor 23 in the housing 10.

As shown in FIG. 7, the first temperature sensor 21 and the second temperature sensor 22 may be located on a side that the speaker 40 faces to the ear 500 of the user, the third temperature sensor 23 may be located on a side that the speaker 40 is away from the ear 500 of the user. In this way, while implementing a core temperature measurement of the user, space in the housing 10 can be effectively used to implement an assembly of a temperature sensor 20.

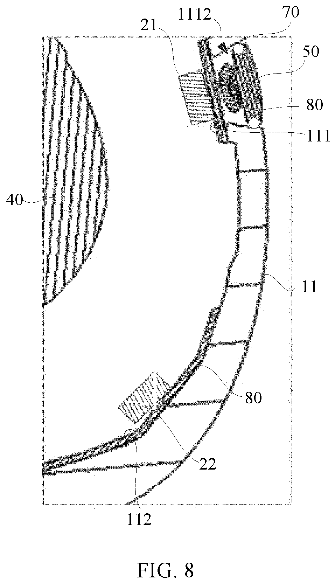

FIG. 8 is an enlarged view of a part A of the earphone in FIG 7.

As shown in FIG. 8, the wearable electronic apparatus 100 may further include a thermally conductive metal member 50, the thermally conductive metal member 50 is embedded in a contact part 1111, and is exposed on an outer surface of the housing 10. In this way, when the wearable electronic apparatus 100 is worn on the ear 500 of the user, the thermally conductive metal member 50 can be directly in contact with the ear 500 of the user, and heat of the user is transferred to a first temperature measurement point 111, so that while determining the core temperature of the user by using the processor 30 based on a thermal equilibrium temperature of the first temperature sensor 21, transfer efficiency of heat of the user transfer to the first temperature measurement point 111 can be accelerated, thereby improving a temperature measurement rate.

For example, the thermally conductive metal member 50 may be made of a material that includes, but is not limited to, a stainless steel member, a copper member, an aluminum member, or another thermal conductive material with low thermal resistance. Because the stainless steel member, the copper member, and the aluminum member have good thermally conductive effects, and also have low thermal resistance, in this embodiment, by a setting of the stainless steel member, the copper member, and the aluminum member, based on an implementation that the heat of the user is transferred to the first temperature measurement point 111, the first temperature sensor 21 is enable to be in contact with the ear 500 by using a low thermal resistance material. In this way, the thermal resistance between the first temperature sensor 21 and the ear 500 can be reduced, and the thermal resistance between the second temperature sensor 22 and the ear 500 is greater than the thermal resistance between the first temperature sensor 21 and the ear 500, so that a temperature differential function of the second temperature sensor 22 relative to the first temperature sensor 21 is implemented.

To further increase the temperature differential function of the second temperature sensor 22 relative to the first temperature sensor 21, in some embodiments, a distance between the second temperature sensor 22 and the eardrum of the user may be greater than a distance between the first temperature sensor 21 and the eardrum of the user. In this way, the thermal resistance between the second temperature sensor 22 and the ear 500 may be further increased to a certain extent to ensure that there is a good temperature differential function of the second temperature sensor 22 relative to the first temperature sensor 21.

To facilitate provision of the thermally conductive metal member 50, as shown in FIG. 8, the contact part 1111 may be provided with a mounting hole 1112 adapted to a structure of the thermally conductive metal member 50, the mounting hole 1112 is connected to a cavity of the housing 10, so that the thermally conductive metal member 50 is embedded in the contact part 1111. The thermally conductive metal member 50 may be fixed to the housing 10 through bonding, interference fit, clamping, threaded connection, or in another manner.

To avoid provision of the mounting hole 1112 affecting a sealing performance of the wearable electronic apparatus 100, in some embodiments, the thermally conductive metal member 50 may be connected to the housing 10 in a sealing manner. The thermally conductive metal member 50 may be connected to the housing 10 through a sealing member 70 in a sealing manner. In this embodiment, as shown in FIG. 8, the sealing member 70 may be located on a peripheral side of the thermally conductive metal member 50, and sealed in a gap of the mounting hole 1112 of the thermally conductive metal member 50. Alternatively, the sealing member 70 may also be located at another position of the housing 10, such as a side that the mounting hole 1112 faces to inside of the housing 10. For example, the sealing member 70 may include but is not limited to a sealing ring. In this way, a sealing performance of the wearable electronic apparatus 100 may be enhanced by provision of the sealing member 70, thereby further improving accuracy for measuring the core temperature.

In some embodiments, as shown in FIG. 8, the first temperature sensor 21 may be located on a side that is of the flexible circuit board 80 and that faces to a center of the housing 10. As a possible implementation, the first temperature sensor 21 is configured to measure a temperature of a position that is of the flexible circuit board 80 and that corresponds to the first temperature sensor 21. At this time, the position that is on the flexible circuit board 80 and that corresponds to the first temperature sensor 21 constitutes the first temperature measurement point 111, so that a temperature of the ear 500 transferred to the first temperature measurement point 111 is indirectly reflected based on a temperature at the first temperature measurement point 111. Internal space of the housing 10 can be effectively used while the body temperature measurement of the user can be implemented.

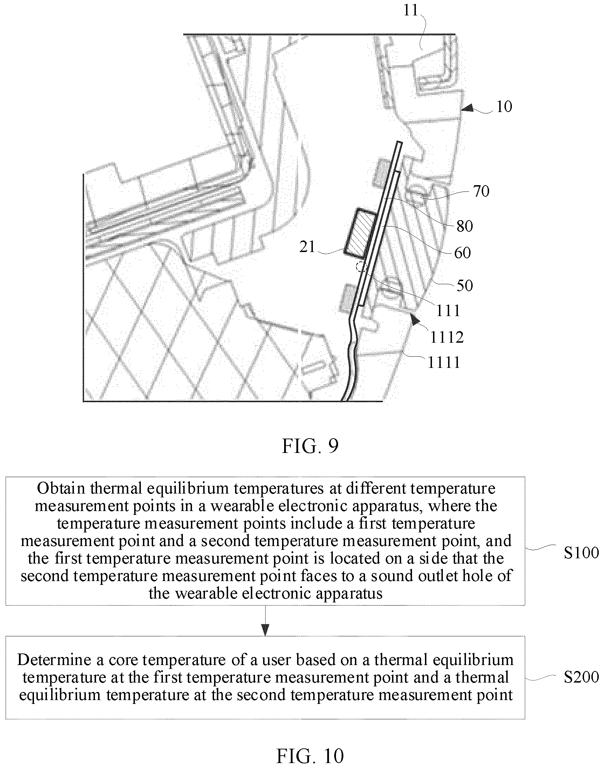

FIG. 9 is a schematic diagram of an assembly of a first temperature sensor in an earphone housing. To facilitate heat absorbed by a thermally conductive metal member 50 to conduct to a first temperature measurement point 111, referring to FIG. 9, a housing 10 has a thermally conductive layer 60. For example, the thermally conductive layer 60 may include but is not limited to a thermally conductive adhesive layer. The thermally conductive layer 60 is fitted between a flexible circuit board 80 and the thermally conductive metal member 50. By provision of the thermally conductive layer 60, heat of the thermally conductive metal member 50 is better and faster transferred to the first temperature measurement point 111, to avoid a loss of heat in a transfer process, so that accuracy for measuring the core temperature can be improved, and due to provision of the thermally conductive adhesive layer, the thermally conductive metal member 50 can be fixed on the flexible circuit board 80, to simplify a structure of a wearable electronic apparatus 100.

Alternatively, in some other embodiments, a first temperature sensor 21 may be located on a side of the flexible circuit board 80 facing to a contact part 1111, and the first temperature sensor 21 is configured to measure a temperature of the contact part 1111. At this time, a part that is of the contact part 1111 and that faces to the first temperature sensor 21 may constitute the first temperature measurement point 111.

In this embodiment, a second temperature sensor 22 may be located on the flexible circuit board 80 the same as the first temperature sensor 21. For example, both the second temperature sensor 22 and the first temperature sensor 21 may be located on a side that is of the flexible circuit board 80 and that faces to a center of the housing 10 (as shown in FIG. 8). As a possible implementation, the second temperature sensor 22 may measure a temperature of a position that is of the flexible circuit board 80 and that corresponds to the second temperature sensor 22. At this time, the position that is on the flexible circuit board 80 and that corresponds to the second temperature sensor 22 constitutes a second temperature measurement point 112, so that a temperature of an ear 500 transferred to the second temperature measurement point 112 is indirectly reflected based on a temperature at the second temperature measurement point 112.

Alternatively, in some embodiments, the second temperature sensor 22 may be located on the flexible circuit board 80 and a side that is opposite to the first temperature sensor 21. For example, the first temperature sensor 21 may be located on a side that is of the flexible circuit board 80 and that faces to a center of the housing 10, the second temperature sensor 22 may be located on a side that is of the flexible circuit board 80 and that faces to the ear 500 of the user, and the second temperature sensor 22 may measure a temperature of the housing 10 such as an earphone housing 11 corresponding to the second temperature sensor 22. At this time, a part that is of the earphone housing 11 and that corresponds to the second temperature sensor 22 may constitute the second temperature measurement point 112. In this way, a temperature at the second temperature measurement point 112 can be measured by using the second temperature sensor 22, and a provision manner of the second temperature sensor 22 in the housing 10 may be more diversified. A third temperature sensor 23 may be located on the side that is of the flexible circuit board 80 and that faces to or is away from the ear 500 of the user, the third temperature sensor 23 may be configured to measure a temperature of a position that is on the flexible circuit board 80 and that corresponds to the third temperature sensor 23. At this time, the position that is of the flexible circuit board 80 and that corresponds to the third temperature sensor 23 constitutes ambient temperature measurement point 121. Alternatively, the third temperature sensor 23 may also measure a temperature of the housing 10 such as an earphone handle housing 12 corresponding to the third temperature sensor 23. At this time, a part that is of the earphone handle housing 12 and that corresponds to the third temperature sensor 23 may constitute the ambient temperature measurement points 121 (as shown in FIG. 7), so that an ambient temperature inside and outside the wearable electronic apparatus 100 may be reflected based on temperatures at the ambient temperature measurement points 121.

It should be noted that, the wearable electronic apparatus 100 may also include a power module, a microphone module, a Bluetooth module and a charging accommodating apparatus, and the like. The power module, the microphone module, and the Bluetooth module may be provided in the housing 10.

Based on the foregoing embodiments, this embodiment of this application also provides a wearable electronic device, the wearable electronic device includes a charging accommodating apparatus and the foregoing wearable electronic apparatus. The wearable electronic apparatus is provided in the charging accommodating apparatus. For example, the charging accommodating apparatus may include but is not limited to a charging accommodating box. In this way, by disposing the charging accommodating apparatus, the wearable electronic apparatus not only can be charged, but the wearable electronic apparatus also can be accommodated.

FIG. 10 is a schematic flowchart of a body temperature measurement method according to an embodiment of this application.

Based on the foregoing embodiment, an embodiment of this application further provides a body temperature measurement method, and the measurement method is applied to the foregoing wearable electronic apparatus 100. As shown in FIG. 10, the measurement method may include: Step S100: Obtain thermal equilibrium temperatures at different temperature measurement points in the wearable electronic apparatus, where the temperature measurement points include a first temperature measurement point and a second temperature measurement point, and the first temperature measurement point is located on a side that the second temperature measurement point faces to a sound outlet hole of the wearable electronic apparatus.

It should be noted that, in this embodiment, when the wearable electronic apparatus 100 is worn on an ear 500 of a user, temperatures at a first temperature measurement point 111 and a second temperature measurement point 112 may be measured by using a first temperature sensor 21 and a second temperature sensor 22 in the wearable electronic apparatus 100 respectively, to obtain thermal equilibrium temperatures at the first temperature measurement point 111 and the second temperature measurement point 112, and to facilitate measurement of body temperature of the user, such as a core temperature.

Step S200: Determine a core temperature of the user based on the thermal equilibrium temperature at the first temperature measurement point and the thermal equilibrium temperature at the second temperature measurement point.

It should be noted that, in this embodiment, after thermal equilibrium temperatures at different temperature measurement points (such as the first temperature measurement point 111 and the second temperature measurement point 112) may be obtained by using a processor 30 in the wearable electronic apparatus 100, a calculation module is controlled to calculate the core temperature of the user based on the thermal equilibrium temperature at the first temperature measurement point 111 and the thermal equilibrium temperature at the second temperature measurement point 112, to determine the core temperature of the user, so that the wearable electronic apparatus 100 can implement a core temperature measurement of the user, and functions of the wearable electronic apparatus 100 can be more diversified.

For the calculation of the core temperature by the calculation module, refer to the foregoing relevant descriptions. Details are not described herein again.

To improve accuracy for measuring a temperature, the temperature measurement points may also include an ambient temperature measurement point 121, where the ambient temperature measurement point 121 may be located on a side that the second temperature measurement point 112 is away from the first temperature measurement point 111. The determining a core temperature of the user based on the thermal equilibrium temperature at the first temperature measurement point and the thermal equilibrium temperature at the second temperature measurement point in step S200 may specifically include:

determining the core temperature of the user based on the thermal equilibrium temperature at the first temperature measurement point 111, the thermal equilibrium temperature at the second temperature measurement point 112, and an ambient temperature at the ambient temperature measurement point 121.

It should be noted that, the processor 30 may control the calculation module to calculate and determine the core temperature of the user based on the foregoing formula 4, the thermal equilibrium temperature at the first temperature measurement point 111 and the second temperature measurement point 112, and the ambient temperature at the ambient temperature measurement point 121. In this way, by provision of the ambient temperature at the ambient temperature measurement point 121, an impact of an external affecting factor, such as an ambient heat source 300, and an impact of thermal power consumption of the wearable electronic apparatus 100, such as a power consumption heat source 400, on the core temperature can be eliminated, and accuracy of the wearable electronic apparatus 100 for a core temperature measurement can be improved.

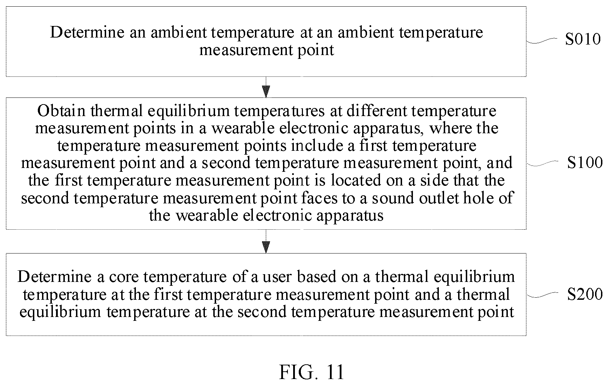

FIG. 11 is a schematic flowchart of another body temperature measurement method according to an embodiment of this application.

As shown in FIG. 11, before obtaining thermal equilibrium temperatures at different temperature measurement points in the wearable electronic apparatus, the measurement method further includes:

Step S010: Determine an ambient temperature at an ambient temperature measurement point. It should be noted that, the ambient temperature at the ambient temperature measurement point 121 can only be obtained by using the third temperature sensor 23. However, the ambient temperature at the ambient temperature measurement point 121 is affected by the ambient temperature measurement point 121 and various scenario factors, and may not accurately reflect temperature in internal and external environments of the wearable electronic apparatus 100. When a thermal equilibrium temperature T_3 at the ambient temperature measurement point 121 is obtained inaccurately, an error in a substitution of an adjustment factor may occur very likely, resulting in a reduction in calculation accuracy, and finally affecting accuracy for measuring a core temperature.

In this embodiment, factors that causes inaccurate obtaining of the ambient temperature at the ambient temperature measurement point 121 are classified into the following types:

First type of factor: The wearable electronic apparatus 100 is affected by body temperature when being put in a pocket of a user, a circuit board, such as a flexible circuit board 80, in a charging scenario is affected by heat, and a finger temperature is affected when a finger of a user is in contact with the temperature measurement point.

Second type of factor: The wearable electronic apparatus 100 is affected by different power consumption in a working scenario.

Third type of factor: A scenario in which the wearable electronic apparatus 100 is affected by the obstruction 600 when an ear 500 is covered by an obstruction 600 such as hair, a hat, and clothing.

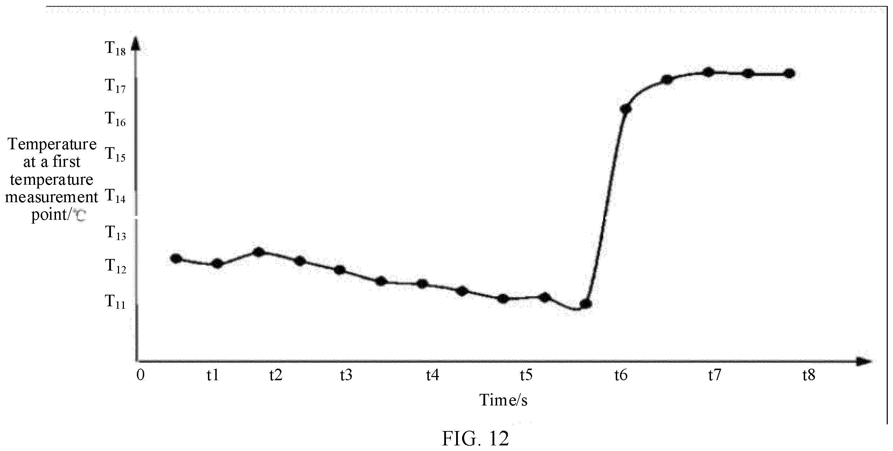

FIG. 12 is a schematic diagram of an impact of a first type of factor on temperatures at temperature measurement points in an earphone.

The horizontal coordinate t1 to t8 represent time, and t1 to t8 increase in sequence. The vertical coordinate represents an ambient temperature at a first temperature measurement point 111, and T11 to T18 increase in sequence. As shown in FIG. 12, during a process from t1 to t2, because a wearable electronic apparatus 100 is in a pocket of a user, during a process of charging the wearable electronic apparatus 100, such as an earphone, in a charging accommodating apparatus, the wearable electronic apparatus 100 is affected by body temperature and charging in the charging accommodating apparatus, so that a temperature at each temperature measurement point, such as an ambient temperature at an ambient temperature measurement point 121, is higher than an actual ambient temperature in which the wearable electronic device is located.

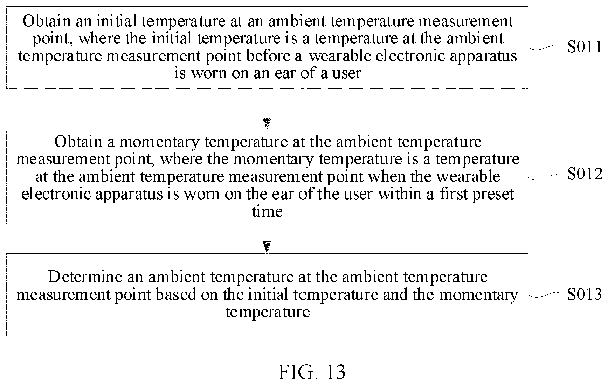

After the wearable electronic apparatus 100 is taken out of the charging accommodating apparatus and the pocket, the temperature at each temperature measurement point gradually decreases as time goes by. At a moment t3, each temperature measurement point gradually approaches the actual ambient temperature in which the wearable electronic device is located. At a moment t6, because a finger of the user is in contacted with a thermally conductive metal member 50 on a surface of the wearable apparatus, at this time, body temperature of the user may be transferred to the temperature measurement point through the thermally conductive metal member 50, causing the temperature at the temperature measurement point to rise rapidly. As a result, finally the temperature at each temperature measurement point, such as the ambient temperature at the ambient temperature measurement point 121, is higher than the actual ambient temperature in which the wearable electronic apparatus 100 is located, and measurement of the ambient temperature at the ambient temperature measurement point 121 may be affected. It can be learned that the first type of factor has a great impact on the ambient temperature at the ambient temperature measurement point 121 in an earphone and a measurement result of a core temperature. FIG. 13 is a schematic flowchart of determining an ambient temperature at an ambient temperature measurement point according to an embodiment of this application.

To eliminate an impact of a first type of factor on the core temperature, as shown in FIG 13, the determining an ambient temperature at an ambient temperature measurement point in step S010 specifically includes:

Step S011: Obtain an initial temperature at the ambient temperature measurement point, where the initial temperature is a temperature at the ambient temperature measurement point before the wearable electronic apparatus is worn on ear of the user;

Step S012: Obtain a momentary temperature at the ambient temperature measurement point, where the momentary temperature is a temperature at the ambient temperature measurement point when the wearable electronic apparatus is worn on the ear of the user within a first preset time; and

Step S013: Determine the ambient temperature at the ambient temperature measurement point based on the initial temperature and the momentary temperature.

It should be noted that, the initial temperature may be measured and obtained by using a third temperature sensor 23 to measure the ambient temperature at the ambient temperature measurement point within a preset time (such as 2s) when a wearable electronic apparatus 100 is accommodated in a charging accommodating apparatus and after the charging accommodating apparatus is opened. The momentary temperature may be measured and obtained by using the third temperature sensor 23 to measure the ambient temperature at the ambient temperature measurement point within a first preset time (such as 1s or 2s) when the wearable electronic apparatus 100 is worn on an ear 500 of the user.

The determining the ambient temperature at the ambient temperature measurement point 121 based on the initial temperature and the momentary temperature in step S013 may specifically include:

using a lower temperature between the initial temperature and the momentary temperature as the ambient temperature at the ambient temperature measurement point 121.

In this way, the ambient temperature at the ambient temperature measurement point 121 can be preliminarily determined based on a change between the initial temperature and the momentary temperature, to eliminate an impact of a first type of factor, such as body temperature in a pocket of the user, charging, and a finger of a user touching, on the ambient temperature at the ambient temperature measurement point 121, and to screen the ambient temperature at the ambient temperature measurement point 121, so that an impact of the first type of factor on measurement of the core temperature of the user can be avoided, and accuracy of the wearable electronic apparatus 100 for a core temperature measurement can be improved.

It should be noted that, in some other embodiments, another method may also be used to determine the ambient temperature at the ambient temperature measurement point 121.

Due to existences of a first temperature sensor 21 and a second temperature sensor 22, a temperature at a first temperature measurement point 111 and a temperature at a second temperature measurement point 112 may also be obtained while obtaining the initial temperature and the momentary temperature at the ambient temperature measurement point 121. In some embodiments, at this time, the temperature at the first temperature measurement point 111 and the temperature at the second temperature measurement point 112 may also be used as an auxiliary condition for determining the ambient temperature at the ambient temperature measurement point 121.

To further improve accuracy for measuring a core temperature, before the determining a core temperature of the user based on the thermal equilibrium temperature at the first temperature measurement point and the thermal equilibrium temperature at the second temperature measurement point in step S200, the measurement method may further include: correcting the ambient temperature at the ambient temperature measurement point 121 based on the ambient temperature at the ambient temperature measurement point 121.

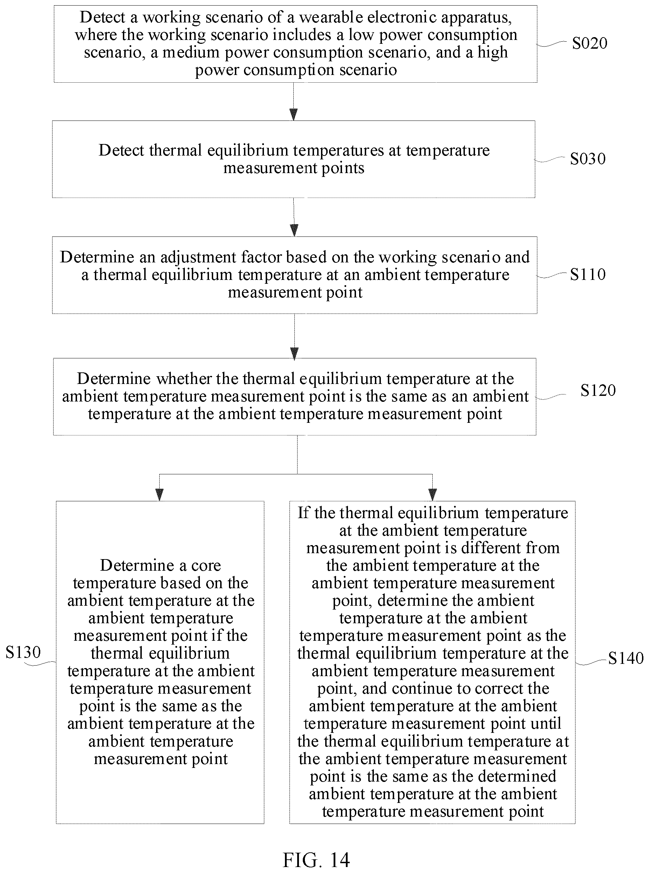

This can facilitate to correct the ambient temperature at the ambient temperature measurement point 121 through the thermal equilibrium temperature at the ambient temperature measurement point 121, and can eliminate an impact of an external influence factor on the core temperature. FIG. 14 is a schematic flowchart of correcting thermal equilibrium temperatures at different temperature measurement points of an earphone.

To eliminate an impact of a second type of factor on core temperature, as shown in FIG 14, the correcting an ambient temperature at an ambient temperature measurement point 121 based on a thermal equilibrium temperature at the ambient temperature measurement point 121 may specifically include:

Step S020: Detect a working scenario of the wearable electronic apparatus, where the working scenario includes a low power consumption scenario, a medium power consumption scenario, and a high power consumption scenario;
Step S030: Measure the thermal equilibrium temperatures at the temperature measurement points; and
Step S110: Determine an adjustment factor based on the working scenario and the thermal equilibrium temperature at the ambient temperature measurement point.

In this way, by using the adjustment factor, an impact of the second type of factor, such as different power consumption working scenarios, on the measurement of the core temperature of a user can be eliminated, and accuracy for measuring the core temperature by the wearable electronic apparatus 100 can be further improved.

It should be noted that, in this embodiment of this application, related operations of step S020 and step S030 may be performed before step S100, and step S110 may be performed after step S100. In this embodiment, a processor 30 can control a register to detect the working scenario of the wearable electronic apparatus 100, to determine adjustment factors a and b based on the monitored thermal equilibrium temperature at the ambient temperature measurement point 121, to apply the adjustment factors a and b to formula 4, and to correct the ambient temperature at the ambient temperature measurement point 121 based on the adjustment factors a and b, so that the impact of the second type of factor on the core temperature is eliminated, and accuracy for measuring the core temperature is improved.

In an equilibrium process of transferring the thermal equilibrium temperature at the ambient temperature measurement point 121, a temperature at the ambient temperature measurement points 121 may be measured by a third temperature sensor 23, to obtain the thermal equilibrium temperature at the ambient temperature measurement point 121. For the method of determining an adjustment factor based on the working scenario and the thermal equilibrium temperature at the ambient temperature measurement point 121, refer to the foregoing relevant descriptions. Details are not described herein again.

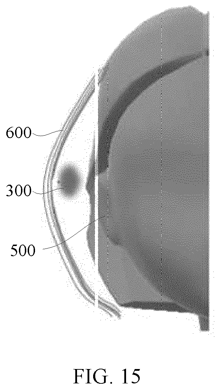

FIG. 15 is a simulated view that an obstruction covers a head phantom.

As shown in FIG. 15, in a third type of factor, an obstruction 600, such as clothing and a hat, covers an ear 500 of a user. Because the obstruction 600 has a certain thermal insulation effect, under the action of the obstruction 600, a local temperature of the ear 500 is higher than an actual ambient temperature in which the user and a wearable electronic apparatus 100, such as an earphone, are located. Therefore, when the wearable electronic apparatus 100 is worn on the ear 500 of the user and the obstruction 600 covers the ear 500, the ambient temperature at the ambient temperature measurement point 121 measured by the third temperature sensor 23 is higher than the actual ambient temperature in which the wearable electronic device is located.

To eliminate an impact of the third type of factor on the ambient temperature at an ambient temperature measurement point 121, as shown in FIG 14, the correcting an ambient temperature at an ambient temperature measurement point 121 based on a thermal equilibrium temperature at the ambient temperature measurement point 121 may specifically include:

Step S120: Determine whether the thermal equilibrium temperature at the ambient temperature measurement point is the same as the ambient temperature at the ambient temperature measurement point;
Step S130: Determine a core temperature based on the ambient temperature at the ambient temperature measurement point 121 if the thermal equilibrium temperature at the ambient temperature measurement point is the same as the ambient temperature at the ambient temperature measurement point; and
Step S140: If the thermal equilibrium temperature at the ambient temperature measurement point is different from the ambient temperature at the ambient temperature measurement point, determine the ambient temperature at the ambient temperature measurement point as the thermal equilibrium temperature at the ambient temperature measurement point, and continue to correct the ambient temperature at the ambient temperature measurement point until the thermal equilibrium temperature at the ambient temperature measurement point is the same as the determined ambient temperature at the ambient temperature measurement point.

It should be noted that, in a thermal equilibrium process, a temperature at each temperature measurement point may be monitored by using a temperature sensor 20. In this way, through a change of the thermal equilibrium temperature at the ambient temperature measurement point 121 relatives to the ambient temperature at the ambient temperature measurement point 121, for example, when the thermal equilibrium temperature at the ambient temperature measurement point is different from the ambient temperature at the ambient temperature measurement point, a scenario that the obstructions 600 of a second type of factor, such as long hair for a woman, clothing, or a hat, covers the ear 500 may be determined. By determining the ambient temperature at the ambient temperature measurement point as the thermal equilibrium temperature at the ambient temperature measurement point, and continuing to correct the ambient temperature at the ambient temperature measurement point until the thermal equilibrium temperature at the ambient temperature measurement point is the same as the determined ambient temperature at the ambient temperature measurement point, an impact of the second type of factor on the core temperature measurement of the user is avoided, and accuracy for measuring the core temperature by the wearable electronic apparatus 100 is further improved.

It should be noted that, in step S140, continuing to correct the ambient temperature at the ambient temperature measurement point may be understood as re-determining the working scenario of the wearable electronic apparatus 100 based on the redetermined ambient temperature at the ambient temperature measurement point 121, and re-determining the adjustment factor and the ambient temperature at the ambient temperature measurement point 121 based on a re-monitored thermal equilibrium temperature at the ambient temperature measurement point 121.

It should be noted that, after correcting the ambient temperature at the ambient temperature measurement point 121, the determining a core temperature of the user based on the thermal equilibrium temperature at the first temperature measurement point 111 and the thermal equilibrium temperature at the second temperature measurement point 112 may specifically include:

determining the core temperature of the user based on the thermal equilibrium temperature at the first temperature measurement point 111, the thermal equilibrium temperature at the second temperature measurement point 112, the ambient temperature at the ambient temperature measurement point 121, and the adjustment factor.

It should be noted that, a processor 30 may control a calculation module to calculate and determine the core temperature of the user based on the foregoing formula 4, the thermal equilibrium temperature at the first temperature measurement point 111 and the second temperature measurement point 112, the ambient temperature at the ambient temperature measurement point 121, and the adjustment factor. In this way, an impact of an external influence factor on the core temperature can be eliminated effectively, and a measurement result of the core temperature performed by the wearable electronic apparatus 100 has high accuracy.

In this way, through the ambient temperature at the ambient temperature measurement point 121 in this embodiment of this application, the thermal equilibrium temperature at the first temperature measurement point 111 and the second temperature measurement point 112 can be corrected, and accuracy for calculating the core temperature is improved.

In some embodiments, factors causing inaccurate obtaining of the ambient temperature at the ambient temperature measurement point 121 may further include:

Fourth type of factor: an impact of a change in an indoor and outdoor scenario and the like.

The following uses the first temperature measurement point 111 as an example, to briefly explain an impact of the fourth type of factor on the ambient temperature at the temperature measurement point.

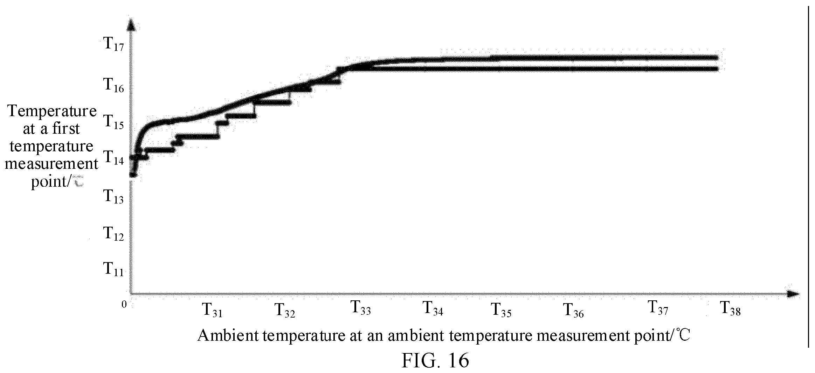

FIG. 16 is a schematic diagram of an impact of a fourth type of factor on ambient temperature at a temperature measurement point in an earphone.

As shown in FIG. 16, when an indoor and outdoor scenario changes, for example, when a user moves from outdoors to indoors, an ambient temperature in which a wearable electronic apparatus 100 is located increases, and an ambient temperature at an ambient temperature measurement point 121 measured by a third temperature sensor 23 is also caused to increase gradually. Successively, when a core temperature is tested, a thermal equilibrium temperature at a first temperature measurement point 111 measured by a first temperature sensor 21 and a thermal equilibrium temperature at a second temperature measurement point 112 measured by a second temperature sensor 22 also change with the ambient temperature at the ambient temperature measurement point 121 accordingly, affecting a measurement result of a core temperature.

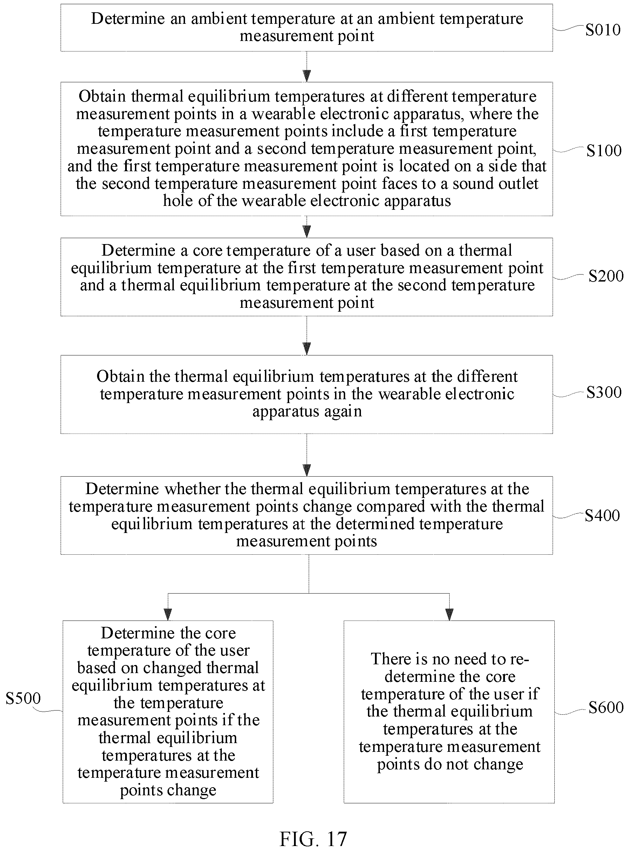

FIG. 17 is a schematic flowchart of still another body temperature measurement method according to an embodiment of this application.

To eliminate an impact of a fourth type of factor on a core temperature measurement, as shown in FIG. 17, after the determining a core temperature of the user based on the thermal equilibrium temperature at the first temperature measurement point and the thermal equilibrium temperature at the second temperature measurement point in step S200, the measurement method may further include:

Step S300: Obtain the thermal equilibrium temperatures at the different temperature measurement points in the wearable electronic apparatus again;

Step S400: Determine whether the thermal equilibrium temperatures at the temperature measurement points change compared with the thermal equilibrium temperatures at the determined temperature measurement points;

Step S500: Determine the core temperature of the user based on changed thermal equilibrium temperatures at the temperature measurement points if the thermal equilibrium temperatures at the temperature measurement points change; and

Step S600: There is no need to re-determine the core temperature of the user if the thermal equilibrium temperatures at the temperature measurement points do not change.

It should be noted that, in step S500, after the thermal equilibrium temperatures at the temperature measurement points change, and in some embodiments, after corresponding data in a calculation module is updated based on a changed thermal equilibrium temperature at the first temperature measurement point 111, a changed thermal equilibrium temperature at the second temperature measurement point 112, and a changed thermal equilibrium temperature at the ambient temperature measurement point 121, the core temperature of the user is calculated and determined. Alternatively, in some other embodiments, after the thermal equilibrium temperatures at the temperature measurement points change, step S020 may be re-performed to re-correct the ambient temperature at the ambient temperature measurement point 121, and after the correction is completed, step S200 is re-performed. In this way, an impact of the fourth type of factor, such as a change in an indoor and outdoor scenario, on the core temperature of the user can be eliminated, and the measurement result of the core temperature can be improved.

In some embodiments, factors causing inaccurate obtaining of the ambient temperature at the ambient temperature measurement point 121 may further include:

Fifth type of factor: A temperature measurement point fails to drop to the ambient temperature when it is put on and off for a short period of time, affecting an identification of a thermal equilibrium temperature.

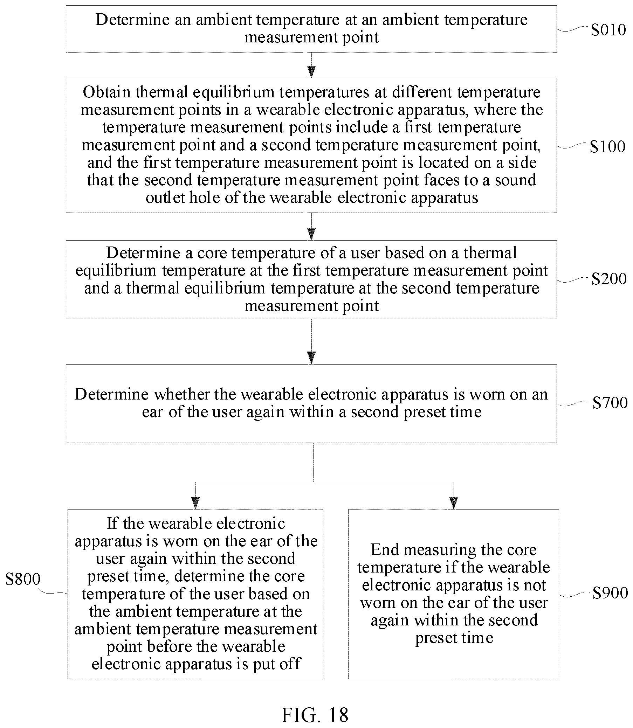

FIG. 18 is a schematic flowchart of yet another body temperature measurement method according to an embodiment of this application.

Because of an impact of a fifth type of factor, when a user put off a wearable electronic apparatus 100 from an ear 500 and wears the wearable electronic apparatus 100 again after a short period of time, it is difficult for a temperature at each temperature measurement point, such as the ambient temperature at the ambient temperature measurement point 121, to drop to an actual ambient temperature for a short period of time (for example, 30s), affecting accuracy for measuring a core temperature.

Therefore, as shown in FIG. 18, after the determining a core temperature of the user based on the thermal equilibrium temperature at the first temperature measurement point and the thermal equilibrium temperature at the second temperature measurement point in step S200, the measurement method may further include:

Step S700: Determine whether the wearable electronic apparatus is worn on the ear of the user again within a second preset time;

Step S800: If the wearable electronic apparatus is worn on the ear of the user again within the second preset time, determine the core temperature of the user based on the ambient temperature at the ambient temperature measurement point before the wearable electronic apparatus is put off; and

Step S900: End measuring the core temperature if the wearable electronic apparatus is not worn on the ear of the user again within the second preset time.

It should be noted that, a wearable electronic device may include two wearable electronic apparatuses 100. The two wearable electronic apparatuses 100 may be worn on a left ear or right ear of the user respectively. When both the two wearable electronic apparatuses 100 are worn on ears 500 of the user, after one of the wearable electronic apparatuses 100 is put off from an ear, and when measuring the core temperature of the put-off wearable electronic apparatus 10 is ended, the core temperature may be measured by another wearable electronic apparatus 100 worn on the ear of the user.

For example, the second preset time may include but is not limited to 30s. In step S800, before the wearable electronic apparatus 100 is put off may be read, based on the ambient temperature at the ambient temperature measurement point 121 determined for a first time or determined during a measuring core temperature at last time, step S020 may be performed again to detect the working scenario of the wearable electronic apparatus, where the working scenario includes a low power consumption scenario, a medium power consumption scenario and a high power consumption scenarios to re-determine the core temperature of the user. In this way, that the ambient temperature at the ambient temperature measurement point 121 in the housing 10 fails to drop to an actual ambient temperature when the wearable electronic apparatus 100 is put on and off for a short period of time can be avoided affecting measurement of the core temperature, so that accuracy of the wearable electronic apparatus 100 for measuring the core temperature can be further improved without re-performing step S010. This can simplify the steps for the wearable electronic apparatus 100 to measure the core temperature of the user.

The following further describes a measurement sequence of the measurement method of the wearable electronic apparatus 100 in this embodiment of this application.

Before measuring the core temperature of the user, the ambient temperature at the ambient temperature measurement point 121 is first determined by using an initial temperature and a momentary temperature at the ambient temperature measurement point 121. Then, the working scenario of the wearable electronic apparatus 100 is determined, and the thermal equilibrium temperature at each temperature measurement point is measured in a thermal equilibrium process. An adjustment factor is determined based on the working scenario of the wearable electronic apparatus 100 and the thermal equilibrium temperature. Next, the ambient temperature at the ambient temperature measurement point 121 is corrected by using the adjustment factor and the thermal equilibrium temperature at the ambient temperature measurement point 121. After the correction is completed, finally the core temperature of the user is calculated based on the measured thermal equilibrium temperature at the first temperature measurement point 111 and the second temperature measurement point 112, and a corrected ambient temperature at the ambient temperature measurement point 121.

That after the core temperature calculation is completed may be understood as an end of a temperature measurement. After the temperature measurement is completed, if the thermal equilibrium temperature at each temperature measurement point is monitored to change, the core temperature of the user is automatically determined again based on a changed thermal equilibrium temperature at each temperature measurement point, so that an automatic and continuous measurement of body temperature of the user by the wearable electronic apparatus 100 is implemented. After detecting that the wearable electronic apparatus 100 is put off from the ear 500 of the user, it is detected whether the wearable electronic apparatus 100 is worn on the ear 500 of the user again within a second preset time. If the wearable electronic apparatus 100 is worn again on the ear 500 of the user within the second preset time, the core temperature of the user is automatically determined again based on the ambient temperature at the ambient temperature measurement point 121 before the wearable electronic apparatus 100 is put off, and if the wearable electronic device is not worn on the ear 500 of the user again within the second preset time, the automatic measurement of the core temperature ends.

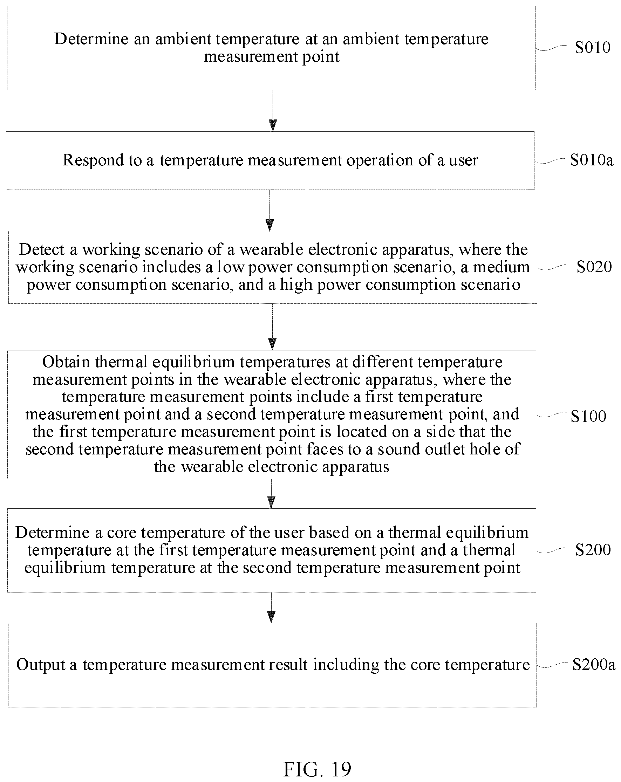

FIG. 19 is a schematic flowchart of yet another body temperature measurement method according to an embodiment of this application.

As shown in FIG. 19, in some embodiments, before obtaining thermal equilibrium temperatures at different temperature measurement points of a housing in the wearable electronic apparatus in step S 100, the body temperature measurement method may further include:

Step S010a: Respond to a temperature measurement operation of the user.

It should be noted that, step S010a may be performed after determining an ambient temperature at an ambient temperature measurement point in step S010 and before detecting a working scenario of the wearable electronic apparatus in step S020. To be specific, in response to the temperature measurement operation of the user, the ambient temperature at the ambient temperature measurement point is already determined in step S010a. In this way, the user can better control a temperature measurement function of the wearable electronic apparatus 100 while not affecting the determining of the ambient temperature at the ambient temperature measurement point. When the user needs to perform the temperature measurement, the temperature measurement function of the wearable electronic apparatus 100 can be started by using a processor 30.

The wearable electronic apparatus 100 may further include a touch sensor. The touch sensor may be located in a housing 10 and electrically connected to the processor 30. A surface of the housing 10 such as the earphone handle housing 12, may form a touch area in an area corresponding to the touch sensor. The temperature measurement operation may include but is not limited to tapping the touch area. The touch sensor may sense the temperature measurement operation of the user. The processor 30 may be configured to respond to the temperature measurement operation of the user when a quantity of the temperature measurement operations of the user sensed by the touch sensor reaches a first preset value. For example, the first preset value may include but is not limited to three times. To be specific, after the user taps the touch area three times, the processor 30 may respond to the temperature measurement operation of the user. After responding to the temperature measurement operation of the user, step S020 and a subsequent operation are performed to determine the core temperature of the user until an automatic measurement of the core temperature is completed.

It should be noted that, when the user taps the touch area again and a quantity of taps reaches a second preset value, the wearable electronic apparatus 100 may turn off the automatic measurement for the body temperature of the user. The second preset value may be the same as the first preset value, or the second preset value may be greater or less than the first preset value. **In** this embodiment, a quantity of the second preset value is not further limited.

**In** some embodiments, when two wearable electronic apparatuses 100 in a wearable electronic device are both worn on ear 500 of the user, tapping touch areas of the two wearable electronic apparatuses 100 at the same time may also be the temperature measurement operation. Alternatively, in some other embodiments, a manner like pressing a button may also be used as the temperature measurement operation to control the temperature measurement function of the wearable electronic apparatus 100. Alternatively, when the wearable electronic apparatus 100 is worn on the ear 500 and is electrically connected to an external electronic device such as a mobile phone, a manner of triggering the mobile phone may also be used as the temperature measurement operation, so that the user controls the temperature measurement function of the wearable electronic apparatus 100 by using the mobile phone. **In** this embodiment, a manner of the temperature measurement operation is not further limited.

As shown in FIG. 19, in some embodiments, after the determining a core temperature of the user based on the thermal equilibrium temperature at the first temperature measurement point and the thermal equilibrium temperature at the second temperature measurement point in step S 100, the body temperature measurement method may further include:

Step S200a: Output a temperature measurement result including the core temperature.

It should be noted that, after the determining the core temperature of the user in step S500 and the determining the core temperature of the user in step S800, step S200a may be performed to output the temperature measurement result including the core temperature. **In** this way, after each time the core temperature of the user is determined, the step of outputting the temperature measurement result is performed, so that the user can learn about the body temperature and a health condition of the user in time.

The temperature measurement result may be directly voice broadcast by using a speaker 40 in the wearable electronic apparatus 100, and the temperature measurement result is output to the user. Alternatively, the measurement result may also be transmitted to an external electronic device connected to the wearable electronic apparatus 100, such as a mobile phone, and directly displayed on the mobile phone, so that the user may obtain the temperature measurement result from the mobile phone.

Alternatively, in some embodiments, while the temperature measurement result is directly voice broadcast by using the speaker 40, the temperature measurement result may also be directly displayed on the mobile phone to avoid the user being unable to accurately receive the voice broadcast from the speaker 40 in a noisy environment and improve accuracy for obtaining a temperature measurement result by the user. In this embodiment, a manner of outputting the temperature measurement result is not further limited.

By provision of a temperature sensor 20 and the processor 30, the wearable electronic apparatus 100 in this embodiment of this application can implement measurement of the core temperature of the user, and diversity of functions of the wearable electronic apparatus 100 can be increased.

It should be noted that, the processor 30 is configured to perform the body temperature measurement method according to any one of the above. By provision of the processor 30, an automatic and continuous measurement of the core temperature of the user can be implemented by using the body temperature measurement method in this embodiment of this application.

The processor 30 may be a central processing unit (Central Processing Unit, CPU for short), and the processor 30 may also be another general-purpose processor, a digital signal processor (Digital Signal Processor, DSP for short), an application specific integrated circuit (Application Specific Integrated Circuit, ASIC for short), or the like. The general-purpose processor may be a microprocessor, or the processor may be any conventional processor or the like. Steps of the methods disclosed with reference to the present invention may be directly performed and completed by a hardware processor, or may be performed and completed by using a combination of hardware and a software module in the processor.

Based on the foregoing embodiments, an embodiment of this application provides a storage medium. The storage medium stores computer-executable instructions. When the computer-executable instructions are executed by a processor, the body temperature measurement method according to any one of the above is implemented. In this way, by provision of the computer-executable instructions stored in the storage medium, the processor is enabled to perform the body temperature measurement method according to embodiments of this application based on the computer-executable instructions, to measure the core temperature of the user.

The storage medium may be implemented by any type of volatile or nonvolatile storage device or a combination thereof, for example, a static random-access memory (SRAM), an electrically erasable programmable read-only memory (EEPROM), an erasable programmable read-only memory (EPROM), a programmable read-only memory (PROM), a read-only memory (ROM), a magnetic memory, a flash memory, a magnetic disk, or an optical disc. The storage medium may be any available medium accessible to a general-purpose or dedicated computer.

In the descriptions of embodiments of this application, it should be noted that, unless specified or limited otherwise, the terms "mounting", "connected", and "connecting" should be understood broadly, for example, which may be a fixed connection, an indirect connection through an intermediary, or internal communication inside two components or an interaction relationship between two components. A person of ordinary skill in the art can understand specific meanings of the foregoing terms in embodiments of this application according to a specific situation.

The terms such as "first", "second", "third", and "fourth" (if any) in the specification and claims of embodiments of this application and in the accompanying drawings are used for distinguishing between similar objects and not necessarily used for describing any particular order or sequence.

**Claims**

**1.** A wearable electronic apparatus (100), comprising a housing (10), a speaker, a processor (30), a temperature sensor (20) located in the housing (10), a thermally conductive metal member (50), a flexible circuit board (80) located inside the housing and thermally conductive layer (60), wherein the housing (10) has a sound outlet hole (113) and a contact part (1111), the temperature sensor (20) comprises a first temperature sensor (21) and a second temperature sensor (22), the first temperature sensor (21) and the second temperature sensor (22) are configured to respectively measure temperatures at different temperature measurement points, wherein both the first temperature sensor (21) and the second temperature sensor (22) are located on the flexible circuit board (80); the second temperature sensor is located on a side of the first temperature sensor opposite to the sound outlet hole and

the processor (30) is configured to determine a core temperature of a user based on a thermal equilibrium temperature corresponding to the first temperature sensor (21) and a thermal equilibrium temperature corresponding to the second temperature sensor (22);

the contact part (1111) is configured to be located in ear (500) of the user and in contact with the ear (500) and the first temperature sensor (21) and the contact part (1111) are disposed corresponding to each other; the thermally conductive metal member (50) is embedded in the contact part (1111) and exposed on an outer surface of the housing (10);

both the first temperature sensor (21) and the second temperature sensor (22) are located on a side that is of the flexible circuit board (80) and faces to a center of the housing (10) and the first temperature sensor (21) is configured to measure a temperature of a position that is on the flexible circuit board (80) and that corresponds to the first temperature sensor (21), and the second temperature sensor (22) is configured to measure a temperature of a position that is on the flexible circuit board (80) and that corresponds to the second temperature sensor (22); the thermally conductive layer (60) is fitted between the flexible circuit board (80) and the thermally conductive metal member (50) on the housing (10).

2. The wearable electronic apparatus (100) according to claim 1, wherein the temperature sensor (20) further comprises a third temperature sensor (23) disposed in the housing (10), the third temperature sensor is located on a side of the second temperature sensor opposite to the first temperature sensor,

the first temperature sensor (21) and the second temperature sensor (22) are located on a side of the speaker (40) that faces to the ear (500) of the user, and the third temperature sensor (23) is located on a side of the speaker (40) away from the ear (500) of the user, and configured to measure an ambient temperature in which the third temperature sensor (23) is located; and

the processor (30) is configured to determine the core temperature of the user based on the thermal equilibrium temperature corresponding to the first temperature sensor (21), the thermal equilibrium temperature corresponding to the second temperature sensor (22), and the ambient temperature.

3. The wearable electronic apparatus (100) according to claim 2, wherein a part that is of the housing (10) and that is located on the third temperature sensor (23) is located outside ear (500) of the user.

4. The wearable electronic apparatus (100) according to claim 2, comprising a calculation module, wherein the calculation module is located in the housing (10), and the processor (30) is configured to control the calculation module to determine the core temperature based on the thermal equilibrium temperature corresponding to the first temperature sensor (21), the thermal equilibrium temperature corresponding to the second temperature sensor (22), and the ambient temperature.

5. The wearable electronic apparatus (100) according to claim 4, wherein the processor (30) is configured to control the calculation module to determine the core temperature based on a formula:

$$T = \frac{R_2(T_1 - T_2 - b \times T_3)}{R_1} + T_1 + a \times T_3,$$

wherein

T is the core temperature, $T_1$ is the thermal equilibrium temperature corresponding to the first temperature sensor (21), $T_2$ is the thermal equilibrium temperature corresponding to the second temperature sensor (22), $T_3$ is the ambient temperature; $R_1$ is equivalent thermal resistance between the first temperature sensor (21) and the second temperature sensor (22) during a heat transfer process, $R_2$ is equivalent thermal resistance between human tissue and the first temperature sensor (21) during a heat transfer process, and a and b are both adjustment factors.

6. The wearable electronic apparatus (100) according to claim 1, wherein thermal resistance between the second temperature sensor (22) and the ear (500) is greater than thermal resistance between the first temperature sensor (21) and the ear (500).

7. The wearable electronic apparatus (100) according to claim 6, wherein the thermally conductive metal member (50) is a stainless steel member, a copper member, or an aluminum member.

8. A body temperature measurement method, wherein the measurement method is applied to the wearable electronic apparatus (100) according to any one of claims 1 to 7, and the measurement method comprises:

    obtaining thermal equilibrium temperatures at different temperature measurement points of a housing (10) in the wearable electronic apparatus (100), wherein the temperature measurement points comprise a first temperature measurement point (111) and a second temperature measurement point (112), the first temperature measuring point being located on a side of the second temperature measuring point facing the sound outlet of the wearable electronic apparatus; and
    determining a core temperature of a user based on a thermal equilibrium temperature at the first temperature measurement point (111) and a thermal equilibrium temperature at the second temperature measurement point (112).

9. The measurement method according to claim 8, wherein the temperature measurement points further comprise an ambient temperature measurement point (121), and the ambient temperature measurement point is located on a side of the second temperature measurement point opposite to the first temperature measurement point; and the determining a core temperature of the user based on the thermal equilibrium temperature at the first temperature measurement point (111) and the thermal equilibrium temperature at the second temperature measurement point (112) specifically comprises:
determining the core temperature of the user based on the thermal equilibrium temperature at the first temperature measurement point (111), the thermal equilibrium temperature at the second temperature measurement point (112), and an ambient temperature at the ambient temperature measurement point (121).

10. The measurement method according to claim 9, wherein before the obtaining thermal equilibrium temperatures at different temperature measurement points of a housing (10) in the wearable electronic apparatus (100), the measurement method further comprises:
determining the ambient temperature at the ambient temperature measurement point (121).

11. The measurement method according to claim 10, wherein the determining the ambient temperature at the ambient temperature measurement point (121) specifically comprises:

    obtaining an initial temperature at the ambient temperature measurement point (121), wherein the initial temperature is a temperature at the ambient temperature measurement point (121) before the wearable electronic apparatus (100) is worn on ear (500) of the user;
    obtaining a momentary temperature at the ambient temperature measurement point (121), wherein the momentary temperature is a temperature at the ambient temperature measurement point (121) when the wearable electronic apparatus (100) is worn on the ear (500) of the user within a first preset time; and
    determining the ambient temperature at the ambient temperature measurement point (121) based on the initial temperature and the momentary temperature.

12. The measurement method according to claim 10, wherein before the determining a core temperature of the user based on the thermal equilibrium temperature at the first temperature measurement point (111) and the thermal equilibrium temperature at the second temperature measurement point (112), the measurement method further comprises:
correcting the ambient temperature at the ambient temperature measurement point (121) based on a thermal equilibrium temperature at the ambient temperature measurement point (121).

13. The measurement method according to claim 11, wherein the correcting the ambient temperature at the ambient temperature measurement point (121) based on a thermal equilibrium temperature at the ambient temperature measurement point (121) specifically comprises:

    detecting a working scenario of the wearable electronic apparatus (100), wherein the working scenario comprises

24

a low power consumption scenario, a medium power consumption scenario, and a high power consumption scenario;

measuring the thermal equilibrium temperatures at the temperature measurement points; and

determining an adjustment factor based on the working scenario and the thermal equilibrium temperature at the ambient temperature measurement point (121).

14. The measurement method according to claim 13, wherein the correcting the ambient temperature at the ambient temperature measurement point (121) based on a thermal equilibrium temperature at the ambient temperature measurement point (121) specifically comprises:

determining whether the thermal equilibrium temperature at the ambient temperature measurement point (121) is the same as the ambient temperature at the ambient temperature measurement point (121);

determining the core temperature based on the ambient temperature at the ambient temperature measurement point (121) if the thermal equilibrium temperature at the ambient temperature measurement point (121) is the same as the ambient temperature at the ambient temperature measurement point (121); and

if the thermal equilibrium temperature at the ambient temperature measurement point (121) is different from the ambient temperature at the ambient temperature measurement point (121), determining the ambient temperature at the ambient temperature measurement point (121) as the thermal equilibrium temperature at the ambient temperature measurement point (121), and continuing to correct the ambient temperature at the ambient temperature measurement point (121) until the thermal equilibrium temperature at the ambient temperature measurement point (121) is the same as the determined ambient temperature at the ambient temperature measurement point (121).

15. A storage medium, wherein the storage medium stores computer-executable instructions, and when the computer-executable instructions are executed by the processor of a wearing electronic apparatus according to any of claims 1-7, the body temperature measurement method according to any one of claims 8 to 14 is implemented.

**Patentansprüche**

1. Tragbare elektronische Vorrichtung (100), umfassend ein Gehäuse (10), einen Lautsprecher, einen Prozessor (30), einen im Gehäuse (10) angeordneten Temperatursensor (20), ein wärmeleitfähiges Metallelement (50), eine im Gehäuse angeordnete flexible Leiterplatte (80) und eine wärmeleitfähige Schicht (60), wobei das Gehäuse (10) eine Schallaustrittsöffnung (113) und einen Kontaktteil (1111) aufweist, wobei der Temperatursensor (20) einen ersten Temperatursensor (21) und einen zweiten Temperatursensor (22) umfasst, wobei der erste Temperatursensor (21) und der zweite Temperatursensor (22) dazu konfiguriert sind, jeweils Temperaturen an verschiedenen Temperaturmesspunkten zu messen, wobei sowohl der erste Temperatursensor (21) als auch der zweite Temperatursensor (22) auf der flexiblen Leiterplatte (80) angeordnet sind; wobei der zweite Temperatursensor auf einer der Schallaustrittsöffnung gegenüberliegenden Seite des ersten Temperatursensors angeordnet ist und

der Prozessor (30) dazu konfiguriert ist, eine Kerntemperatur eines Benutzers basierend auf einer dem ersten Temperatursensor (21) entsprechenden thermischen Gleichgewichtstemperatur und einer dem zweiten Temperatursensor (22) entsprechenden thermischen Gleichgewichtstemperatur zu bestimmen;

der Kontaktteil (1111) dazu konfiguriert ist, im Ohr (500) des Benutzers angeordnet zu sein und in Kontakt mit dem Ohr (500) zu stehen, und der erste Temperatursensor (21) und der Kontaktteil (1111) einander entsprechend angeordnet sind; wobei das wärmeleitfähige Metallelement (50) in den Kontaktteil (1111) eingebettet und an einer Außenfläche des Gehäuses (10) freigelegt ist;

wobei sowohl der erste Temperatursensor (21) als auch der zweite Temperatursensor (22) auf einer Seite der flexiblen Leiterplatte (80) angeordnet sind, die einem Zentrum des Gehäuses (10) zugewandt ist, und der erste Temperatursensor (21) dazu konfiguriert ist, eine Temperatur einer Position auf der flexiblen Leiterplatte (80) zu messen, die dem ersten Temperatursensor (21) entspricht, und der zweite Temperatursensor (22) dazu konfiguriert ist, eine Temperatur einer Position auf der flexiblen Leiterplatte (80) zu messen, die dem zweiten Temperatursensor (22) entspricht; wobei die wärmeleitfähige Schicht (60) zwischen der flexiblen Leiterplatte (80) und dem wärmeleitfähigen Metallelement (50) am Gehäuse (10) eingepasst ist.

2. Tragbare elektronische Vorrichtung (100) nach Anspruch 1, wobei der Temperatursensor (20) ferner einen dritten Temperatursensor (23) umfasst, der im Gehäuse (10) angeordnet ist, wobei der dritte Temperatursensor auf einer dem ersten Temperatursensor gegenüberliegenden Seite des zweiten Temperatursensors angeordnet ist,

der erste Temperatursensor (21) und der zweite Temperatursensor (22) sind auf einer Seite des Lautsprechers (40) angeordnet, die dem Ohr (500) des Benutzers zugewandt ist, und der dritte Temperatursensor (23) ist auf einer Seite des Lautsprechers (40) angeordnet, die vom Ohr (500) des Benutzers abgewandt ist, und dazu konfiguriert, eine Umgebungstemperatur zu messen, in der sich der dritte Temperatursensor (23) befindet; und der Prozessor (30) ist dazu konfiguriert, die Kerntemperatur des Benutzers basierend auf der thermischen Gleichgewichtstemperatur, die dem ersten Temperatursensor (21) entspricht, der thermischen Gleichgewichtstemperatur, die dem zweiten Temperatursensor (22) entspricht, und der Umgebungstemperatur zu bestimmen.

3. Die tragbare elektronische Vorrichtung (100) nach Anspruch 2, wobei ein Teil des Gehäuses (10), das sich an dem dritten Temperatursensor (23) befindet, außerhalb des Ohrs (500) des Benutzers angeordnet ist.

4. Die tragbare elektronische Vorrichtung (100) nach Anspruch 2, umfassend ein Berechnungsmodul, wobei das Berechnungsmodul in dem Gehäuse (10) angeordnet ist und der Prozessor (30) dazu konfiguriert ist, das Berechnungsmodul zu steuern, um die Kerntemperatur basierend auf der thermischen Gleichgewichtstemperatur, die dem ersten Temperatursensor (21) entspricht, der thermischen Gleichgewichtstemperatur, die dem zweiten Temperatursensor (22) entspricht, und der Umgebungstemperatur zu bestimmen.

5. Die tragbare elektronische Vorrichtung (100) nach Anspruch 4, wobei der Prozessor (30) dazu konfiguriert ist, das Berechnungsmodul zu steuern, um die Kerntemperatur basierend auf einer Formel zu bestimmen:

$$T = \frac{R_2(T_1 - T_2 - b \times T_3)}{R_1} + T_1 + a \times T_3,$$

wobei
T die Kerntemperatur ist, $T_1$ die thermische Gleichgewichtstemperatur ist, die dem ersten Temperatursensor (21) entspricht, $T_2$ die thermische Gleichgewichtstemperatur ist, die dem zweiten Temperatursensor (22) entspricht, $T_3$ die Umgebungstemperatur ist; $R_1$ der äquivalente Wärmewiderstand zwischen dem ersten Temperatursensor (21) und dem zweiten Temperatursensor (22) während eines Wärmeübertragungsprozesses ist, $R_2$ der äquivalente Wärmewiderstand zwischen menschlichem Gewebe und dem ersten Temperatursensor (21) während eines Wärmeübertragungsprozesses ist und a sowie b beide Anpassungsfaktoren sind.

6. Die tragbare elektronische Vorrichtung (100) nach Anspruch 1, wobei der Wärmewiderstand zwischen dem zweiten Temperatursensor (22) und dem Ohr (500) größer ist als der Wärmewiderstand zwischen dem ersten Temperatursensor (21) und dem Ohr (500).

7. Die tragbare elektronische Vorrichtung (100) nach Anspruch 6, wobei das wärmeleitende Metallelement (50) ein Edelstahlelement, ein Kupferelement oder ein Aluminiumelement ist.

8. Verfahren zur Messung der Körpertemperatur, wobei das Messverfahren auf die tragbare elektronische Vorrichtung (100) nach einem der Ansprüche 1 bis 7 angewendet wird und das Messverfahren umfasst:

Ermitteln von thermischen Gleichgewichtstemperaturen an verschiedenen Temperaturmesspunkten eines Gehäuses (10) der tragbaren elektronischen Vorrichtung (100), wobei die Temperaturmesspunkte einen ersten Temperaturmesspunkt (111) und einen zweiten Temperaturmesspunkt (112) umfassen, wobei der erste Temperaturmesspunkt auf einer dem Schallauslass der tragbaren elektronischen Vorrichtung zugewandten Seite des zweiten Temperaturmesspunkts angeordnet ist; und
Bestimmen einer Kerntemperatur eines Benutzers basierend auf einer thermischen Gleichgewichtstemperatur am ersten Temperaturmesspunkt (111) und einer thermischen Gleichgewichtstemperatur am zweiten Temperaturmesspunkt (112).

9. Messverfahren nach Anspruch 8, wobei die Temperaturmesspunkte ferner einen Umgebungstemperaturmesspunkt (121) umfassen und der Umgebungstemperaturmesspunkt auf einer dem ersten Temperaturmesspunkt gegenüberliegenden Seite des zweiten Temperaturmesspunkts angeordnet ist; und
das Bestimmen einer Kerntemperatur des Benutzers basierend auf der thermischen Gleichgewichtstemperatur am ersten Temperaturmesspunkt (111) und der thermischen Gleichgewichtstemperatur am zweiten Temperaturmesspunkt (112) umfasst insbesondere:
Bestimmen der Kerntemperatur des Benutzers basierend auf der thermischen Gleichgewichtstemperatur am ersten Temperaturmesspunkt (111), der thermischen Gleichgewichtstemperatur am zweiten Temperaturmesspunkt (112) und einer Umgebungstemperatur am Umgebungstemperaturmesspunkt (121).

10. Messverfahren nach Anspruch 9, wobei das Messverfahren vor dem Ermitteln von thermischen Gleichgewichtstemperaturen an verschiedenen Temperaturmesspunkten eines Gehäuses (10) in der tragbaren elektronischen Vorrichtung (100) ferner umfasst:
Bestimmen der Umgebungstemperatur am Umgebungstemperaturmesspunkt (121).

11. Messverfahren nach Anspruch 10, wobei das Bestimmen der Umgebungstemperatur am Umgebungstemperaturmesspunkt (121) insbesondere umfasst:

Ermitteln einer Anfangstemperatur am Umgebungstemperaturmesspunkt (121), wobei die Anfangstemperatur eine Temperatur am Umgebungstemperaturmesspunkt (121) ist, bevor die tragbare elektronische Vorrichtung (100) am Ohr (500) des Benutzers getragen wird;
Ermitteln einer momentanen Temperatur am Umgebungstemperatur-Messpunkt (121), wobei die momentane Temperatur eine Temperatur am Umgebungstemperatur-Messpunkt (121) ist, wenn die tragbare elektronische Vorrichtung (100) innerhalb einer ersten voreingestellten Zeit am Ohr (500) des Benutzers getragen wird; und
Bestimmen der Umgebungstemperatur am Umgebungstemperatur-Messpunkt (121) basierend auf der Anfangstemperatur und der momentanen Temperatur.

12. Messverfahren nach Anspruch 10, wobei das Messverfahren vor dem Bestimmen einer Kerntemperatur des Benutzers basierend auf der thermischen Gleichgewichtstemperatur am ersten Temperaturmesspunkt (111) und der thermischen Gleichgewichtstemperatur am zweiten Temperaturmesspunkt (112) ferner umfasst:
Korrigieren der Umgebungstemperatur am Umgebungstemperatur-Messpunkt (121) basierend auf einer thermischen Gleichgewichtstemperatur am Umgebungstemperatur-Messpunkt (121).

13. Messverfahren nach Anspruch 11, wobei das Korrigieren der Umgebungstemperatur am Umgebungstemperatur-Messpunkt (121) basierend auf einer thermischen Gleichgewichtstemperatur am Umgebungstemperatur-Messpunkt (121) spezifisch umfasst:

Erfassen eines Arbeitsszenarios der tragbaren elektronischen Vorrichtung (100), wobei das Arbeitsszenario ein Szenario mit niedrigem Stromverbrauch, ein Szenario mit mittlerem Stromverbrauch und ein Szenario mit hohem Stromverbrauch umfasst;
Messen der thermischen Gleichgewichtstemperaturen an den Temperaturmesspunkten; und
Bestimmen eines Anpassungsfaktors basierend auf dem Arbeitsszenario und der thermischen Gleichgewichtstemperatur am Umgebungstemperatur-Messpunkt (121).

14. Messverfahren nach Anspruch 13, wobei das Korrigieren der Umgebungstemperatur am Umgebungstemperatur-Messpunkt (121) basierend auf einer thermischen Gleichgewichtstemperatur am Umgebungstemperatur-Messpunkt (121) spezifisch umfasst:

Bestimmen, ob die thermische Gleichgewichtstemperatur am Umgebungstemperatur-Messpunkt (121) dieselbe ist wie die Umgebungstemperatur am Umgebungstemperatur-Messpunkt (121);
Bestimmen der Kerntemperatur basierend auf der Umgebungstemperatur am Umgebungstemperatur-Messpunkt (121), wenn die thermische Gleichgewichtstemperatur am Umgebungstemperatur-Messpunkt (121) dieselbe ist wie die Umgebungstemperatur am Umgebungstemperatur-Messpunkt (121); und
falls die thermische Gleichgewichtstemperatur am Umgebungstemperaturmesspunkt (121) von der Umgebungstemperatur am Umgebungstemperaturmesspunkt (121) abweicht, Bestimmen der Umgebungstemperatur am Umgebungstemperaturmesspunkt (121) als die thermische Gleichgewichtstemperatur am Umgebungstemperaturmesspunkt (121) und Fortsetzen der Korrektur der Umgebungstemperatur am Umgebungstemperaturmesspunkt (121), bis die thermische Gleichgewichtstemperatur am Umgebungstemperaturmesspunkt (121) mit der bestimmten Umgebungstemperatur am Umgebungstemperaturmesspunkt (121) übereinstimmt.

15. Speichermedium, wobei das Speichermedium computerausführbare Anweisungen speichert, und wenn die computerausführbaren Anweisungen durch den Prozessor einer tragbaren elektronischen Vorrichtung nach einem der Ansprüche 1-7 ausgeführt werden, das Körpertemperaturmessverfahren nach einem der Ansprüche 8 bis 14 implementiert wird.

**Revendications**

1. Appareil électronique portable (100), comprenant un boîtier (10), un haut-parleur, un processeur (30), un capteur de température (20) situé dans le boîtier (10), un élément métallique thermoconducteur (50), une carte de circuit imprimé flexible (80) située à l'intérieur du boîtier et une couche thermoconductrice (60), dans lequel le boîtier (10) présente un trou de sortie de son (113) et une partie de contact (1111), le capteur de température (20) comprend un premier capteur de température (21) et un deuxième capteur de température (22), le premier capteur de température (21) et le deuxième capteur de température (22) sont configurés pour mesurer respectivement des températures au niveau de différents points de mesure de température, dans lequel à la fois le premier capteur de température (21) et le deuxième capteur de température (22) sont situés sur la carte de circuit imprimé flexible (80) ; le deuxième capteur de température est situé sur un côté du premier capteur de température opposé au trou de sortie de son et

   le processeur (30) est configuré pour déterminer une température centrale d'un utilisateur sur la base d'une température d'équilibre thermique correspondant au premier capteur de température (21) et d'une température d'équilibre thermique correspondant au deuxième capteur de température (22) ;
   la partie de contact (1111) est configurée pour être située dans l'oreille (500) de l'utilisateur et en contact avec l'oreille (500), et le premier capteur de température (21) et la partie de contact (1111) sont disposés en correspondance l'un avec l'autre ; l'élément métallique thermoconducteur (50) est intégré dans la partie de contact (1111) et exposé sur une surface extérieure du boîtier (10) ;
   à la fois le premier capteur de température (21) et le deuxième capteur de température (22) sont situés sur un côté qui est celui de la carte de circuit imprimé flexible (80) et fait face à un centre du boîtier (10), et le premier capteur de température (21) est configuré pour mesurer une température d'une position qui est sur la carte de circuit imprimé flexible (80) et qui correspond au premier capteur de température (21), et le deuxième capteur de température (22) est configuré pour mesurer une température d'une position qui est sur la carte de circuit imprimé flexible (80) et qui correspond au deuxième capteur de température (22) ; la couche thermoconductrice (60) est montée entre la carte de circuit imprimé flexible (80) et l'élément métallique thermoconducteur (50) sur le boîtier (10).

2. Appareil électronique portable (100) selon la revendication 1, dans lequel le capteur de température (20) comprend en outre un troisième capteur de température (23) disposé dans le boîtier (10), le troisième capteur de température est situé sur un côté du deuxième capteur de température opposé au premier capteur de température,

   le premier capteur de température (21) et le deuxième capteur de température (22) sont situés sur un côté du haut-parleur (40) qui fait face à l'oreille (500) de l'utilisateur, et le troisième capteur de température (23) est situé sur un côté du haut-parleur (40) opposé à l'oreille (500) de l'utilisateur, et configuré pour mesurer une température ambiante dans laquelle le troisième capteur de température (23) est situé ; et
   le processeur (30) est configuré pour déterminer la température centrale de l'utilisateur sur la base de la température d'équilibre thermique correspondant au premier capteur de température (21), de la température d'équilibre thermique correspondant au deuxième capteur de température (22), et de la température ambiante.

3. L'appareil électronique portable (100) selon la revendication 2, dans lequel une partie qui appartient au boîtier (10) et qui est située sur le troisième capteur de température (23) est située à l'extérieur de l'oreille (500) de l'utilisateur.

4. L'appareil électronique portable (100) selon la revendication 2, comprenant un module de calcul, dans lequel le module de calcul est situé dans le boîtier (10), et le processeur (30) est configuré pour commander le module de calcul afin de déterminer la température centrale sur la base de la température d'équilibre thermique correspondant au premier capteur de température (21), de la température d'équilibre thermique correspondant au deuxième capteur de température (22), et de la température ambiante.

5. L'appareil électronique portable (100) selon la revendication 4, dans lequel le processeur (30) est configuré pour commander le module de calcul afin de déterminer la température centrale sur la base d'une formule :

$$T = \frac{R_2(T_1 - T_2 - b \times T_3)}{R_1} + T_1 + a \times T_3,$$

dans laquelle
T est la température centrale, $T_1$ est la température d'équilibre thermique correspondant au premier capteur de

température (21), $T_2$ est la température d'équilibre thermique correspondant au deuxième capteur de température (22), $T_3$ est la température ambiante ; $R_1$ est la résistance thermique équivalente entre le premier capteur de température (21) et le deuxième capteur de température (22) pendant un processus de transfert de chaleur, $R_2$ est la résistance thermique équivalente entre le tissu humain et le premier capteur de température (21) pendant un processus de transfert de chaleur, et a et b sont tous deux des facteurs d'ajustement.

6. L'appareil électronique portable (100) selon la revendication 1, dans lequel la résistance thermique entre le deuxième capteur de température (22) et l'oreille (500) est supérieure à la résistance thermique entre le premier capteur de température (21) et l'oreille (500).

7. L'appareil électronique portable (100) selon la revendication 6, dans lequel l'élément métallique thermoconducteur (50) est un élément en acier inoxydable, un élément en cuivre, ou un élément en aluminium.

8. Procédé de mesure de la température corporelle, dans lequel le procédé de mesure est appliqué à l'appareil électronique portable (100) selon l'une quelconque des revendications 1 à 7, et le procédé de mesure comprend :

l'obtention de températures d'équilibre thermique en différents points de mesure de température d'un boîtier (10) dans l'appareil électronique portable (100), dans lequel les points de mesure de température comprennent un premier point de mesure de température (111) et un second point de mesure de température (112), le premier point de mesure de température étant situé sur un côté du second point de mesure de température faisant face à la sortie de son de l'appareil électronique portable ; et
la détermination d'une température centrale d'un utilisateur basée sur une température d'équilibre thermique au premier point de mesure de température (111) et une température d'équilibre thermique au second point de mesure de température (112).

9. Procédé de mesure selon la revendication 8, dans lequel les points de mesure de température comprennent en outre un point de mesure de température ambiante (121), et le point de mesure de température ambiante est situé sur un côté du second point de mesure de température opposé au premier point de mesure de température ; et
la détermination d'une température centrale de l'utilisateur basée sur la température d'équilibre thermique au premier point de mesure de température (111) et la température d'équilibre thermique au second point de mesure de température (112) comprend spécifiquement :
la détermination de la température centrale de l'utilisateur basée sur la température d'équilibre thermique au premier point de mesure de température (111), la température d'équilibre thermique au second point de mesure de température (112), et une température ambiante au point de mesure de température ambiante (121).

10. Procédé de mesure selon la revendication 9, dans lequel avant l'obtention des températures d'équilibre thermique en différents points de mesure de température d'un boîtier (10) dans l'appareil électronique portable (100), le procédé de mesure comprend en outre :
la détermination de la température ambiante au point de mesure de température ambiante (121).

11. Procédé de mesure selon la revendication 10, dans lequel la détermination de la température ambiante au point de mesure de température ambiante (121) comprend spécifiquement :

l'obtention d'une température initiale au point de mesure de température ambiante (121), dans lequel la température initiale est une température au point de mesure de température ambiante (121) avant que l'appareil électronique portable (100) ne soit porté sur l'oreille (500) de l'utilisateur ;
l'obtention d'une température momentanée au point de mesure de la température ambiante (121), dans laquelle la température momentanée est une température au point de mesure de la température ambiante (121) lorsque l'appareil électronique portable (100) est porté sur l'oreille (500) de l'utilisateur au cours d'une première période prédéfinie ; et
la détermination de la température ambiante au point de mesure de la température ambiante (121) sur la base de la température initiale et de la température momentanée.

12. Procédé de mesure selon la revendication 10, dans lequel, avant la détermination d'une température centrale de l'utilisateur sur la base de la température d'équilibre thermique au premier point de mesure de température (111) et de la température d'équilibre thermique au deuxième point de mesure de température (112), le procédé de mesure comprend en outre :
la correction de la température ambiante au point de mesure de la température ambiante (121) sur la base d'une température d'équilibre thermique au point de mesure de la température ambiante (121).

13. Procédé de mesure selon la revendication 11, dans lequel la correction de la température ambiante au point de mesure de la température ambiante (121) sur la base d'une température d'équilibre thermique au point de mesure de la température ambiante (121) comprend spécifiquement :

la détection d'un scénario de fonctionnement de l'appareil électronique portable (100), dans lequel le scénario de fonctionnement comprend un scénario à faible consommation d'énergie, un scénario à consommation d'énergie moyenne et un scénario à consommation d'énergie élevée ;
la mesure des températures d'équilibre thermique aux points de mesure de température ; et
la détermination d'un facteur d'ajustement sur la base du scénario de fonctionnement et de la température d'équilibre thermique au point de mesure de la température ambiante (121).

14. Procédé de mesure selon la revendication 13, dans lequel la correction de la température ambiante au point de mesure de la température ambiante (121) sur la base d'une température d'équilibre thermique au point de mesure de la température ambiante (121) comprend spécifiquement :

la détermination de si la température d'équilibre thermique au point de mesure de la température ambiante (121) est identique à la température ambiante au point de mesure de la température ambiante (121) ;
la détermination de la température centrale sur la base de la température ambiante au point de mesure de la température ambiante (121) si la température d'équilibre thermique au point de mesure de la température ambiante (121) est identique à la température ambiante au point de mesure de la température ambiante (121) ; et si la température d'équilibre thermique au point de mesure de la température ambiante (121) est différente de la température ambiante au point de mesure de la température ambiante (121), déterminer la température ambiante au point de mesure de la température ambiante (121) comme étant la température d'équilibre thermique au point de mesure de la température ambiante (121), et continuer à corriger la température ambiante au point de mesure de la température ambiante (121) jusqu'à ce que la température d'équilibre thermique au point de mesure de la température ambiante (121) soit identique à la température ambiante déterminée au point de mesure de la température ambiante (121).

15. Support de stockage, dans lequel le support de stockage stocke des instructions exécutables par ordinateur, et lorsque les instructions exécutables par ordinateur sont exécutées par le processeur d'un appareil électronique portable selon l'une quelconque des revendications 1 à 7, le procédé de mesure de la température corporelle selon l'une quelconque des revendications 8 à 14 est mis en œuvre.

FIG. 1

FIG. 2

FIG. 3

22    21    200

| T2 | R1 | T1 | R2 | T |

Direction of heat transfer

FIG. 4

300

T3

21    200

| T2 | R1 | T1 | R2 | T |

Direction of heat transfer

22

FIG. 5

FIG. 6

FIG. 7

FIG. 8

11

10

70

80

21 60

50

111

1112

1111

FIG. 9

| Obtain thermal equilibrium temperatures at different temperature measurement points in a wearable electronic apparatus, where the temperature measurement points include a first temperature measurement point and a second temperature measurement point, and the first temperature measurement point is located on a side that the second temperature measurement point faces to a sound outlet hole of the wearable electronic apparatus | S100 |

| Determine a core temperature of a user based on a thermal equilibrium temperature at the first temperature measurement point and a thermal equilibrium temperature at the second temperature measurement point | S200 |

FIG. 10

| Determine an ambient temperature at an ambient temperature measurement point | S010 |

| Obtain thermal equilibrium temperatures at different temperature measurement points in a wearable electronic apparatus, where the temperature measurement points include a first temperature measurement point and a second temperature measurement point, and the first temperature measurement point is located on a side that the second temperature measurement point faces to a sound outlet hole of the wearable electronic apparatus | S100 |

| Determine a core temperature of a user based on a thermal equilibrium temperature at the first temperature measurement point and a thermal equilibrium temperature at the second temperature measurement point | S200 |

FIG. 11

FIG. 12

Obtain an initial temperature at an ambient temperature measurement point, where the initial temperature is a temperature at the ambient temperature measurement point before a wearable electronic apparatus is worn on an ear of a user ⟋ S011

Obtain a momentary temperature at the ambient temperature measurement point, where the momentary temperature is a temperature at the ambient temperature measurement point when the wearable electronic apparatus is worn on the ear of the user within a first preset time ⟋ S012

Determine an ambient temperature at the ambient temperature measurement point based on the initial temperature and the momentary temperature ⟋ S013

FIG. 13

Detect a working scenario of a wearable electronic apparatus, where the working scenario includes a low power consumption scenario, a medium power consumption scenario, and a high power consumption scenario — S020

Detect thermal equilibrium temperatures at temperature measurement points — S030

Determine an adjustment factor based on the working scenario and a thermal equilibrium temperature at an ambient temperature measurement point — S110

Determine whether the thermal equilibrium temperature at the ambient temperature measurement point is the same as an ambient temperature at the ambient temperature measurement point — S120

S130 — Determine a core temperature based on the ambient temperature at the ambient temperature measurement point if the thermal equilibrium temperature at the ambient temperature measurement point is the same as the ambient temperature at the ambient temperature measurement point

If the thermal equilibrium temperature at the ambient temperature measurement point is different from the ambient temperature at the ambient temperature measurement point, determine the ambient temperature at the ambient temperature measurement point as the thermal equilibrium temperature at the ambient temperature measurement point, and continue to correct the ambient temperature at the ambient temperature measurement point until the thermal equilibrium temperature at the ambient temperature measurement point is the same as the determined ambient temperature at the ambient temperature measurement point — S140

FIG. 14

FIG. 15

FIG. 16

Determine an ambient temperature at an ambient temperature measurement point ⌐S010

Obtain thermal equilibrium temperatures at different temperature measurement points in a wearable electronic apparatus, where the temperature measurement points include a first temperature measurement point and a second temperature measurement point, and the first temperature measurement point is located on a side that the second temperature measurement point faces to a sound outlet hole of the wearable electronic apparatus ⌐S100

Determine a core temperature of a user based on a thermal equilibrium temperature at the first temperature measurement point and a thermal equilibrium temperature at the second temperature measurement point ⌐S200

Obtain the thermal equilibrium temperatures at the different temperature measurement points in the wearable electronic apparatus again ⌐S300

Determine whether the thermal equilibrium temperatures at the temperature measurement points change compared with the thermal equilibrium temperatures at the determined temperature measurement points ⌐S400

S500 — Determine the core temperature of the user based on changed thermal equilibrium temperatures at the temperature measurement points if the thermal equilibrium temperatures at the temperature measurement points change

There is no need to re-determine the core temperature of the user if the thermal equilibrium temperatures at the temperature measurement points do not change ⌐S600

FIG. 17

Determine an ambient temperature at an ambient temperature measurement point —— S010

Obtain thermal equilibrium temperatures at different temperature measurement points in a wearable electronic apparatus, where the temperature measurement points include a first temperature measurement point and a second temperature measurement point, and the first temperature measurement point is located on a side that the second temperature measurement point faces to a sound outlet hole of the wearable electronic apparatus —— S100

Determine a core temperature of a user based on a thermal equilibrium temperature at the first temperature measurement point and a thermal equilibrium temperature at the second temperature measurement point —— S200

Determine whether the wearable electronic apparatus is worn on an ear of the user again within a second preset time —— S700

S800 —— If the wearable electronic apparatus is worn on the ear of the user again within the second preset time, determine the core temperature of the user based on the ambient temperature at the ambient temperature measurement point before the wearable electronic apparatus is put off

End measuring the core temperature if the wearable electronic apparatus is not worn on the ear of the user again within the second preset time —— S900

FIG. 18

Determine an ambient temperature at an ambient temperature measurement point — S010

Respond to a temperature measurement operation of a user — S010a

Detect a working scenario of a wearable electronic apparatus, where the working scenario includes a low power consumption scenario, a medium power consumption scenario, and a high power consumption scenario — S020

Obtain thermal equilibrium temperatures at different temperature measurement points in the wearable electronic apparatus, where the temperature measurement points include a first temperature measurement point and a second temperature measurement point, and the first temperature measurement point is located on a side that the second temperature measurement point faces to a sound outlet hole of the wearable electronic apparatus — S100

Determine a core temperature of the user based on a thermal equilibrium temperature at the first temperature measurement point and a thermal equilibrium temperature at the second temperature measurement point — S200

Output a temperature measurement result including the core temperature — S200a

FIG. 19

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202210182919
- US 2019117155 A1